# EUROPEAN PATENT APPLICATION

(11) **EP 4 592 379 A1**
(43) Date of publication of application: **30.07.2025**
(21) Application number: 23868242.1
(22) Date of filing: 21.09.2023
(51) Int. Cl.: C12M 1/00, G01N 33/53

(54) **METHOD FOR FORMING MICRODROPLETS**

(30) Priority: 22.09.2022 JP 2022151862
(71) Applicant: Craif Inc., Tokyo 113-0034 (JP)
(72) Inventor: YASUI, Takao, Nagoya-shi, Aichi 464-8601 (JP); BABA, Yoshinobu, Nagoya-shi, Aichi 464-8601 (JP)
(74) Representative: Michalski Hüttermann & Partner Patentanwälte mbB
(86) International application number: PCT/JP2023/034231
(87) International publication number: WO 2024/063121

(57) **Abstract**

One embodiment of the present disclosure provides a method for processing an aqueous solution / a method for forming microdroplets, the method including: providing a substrate/device/flow channel having a surface including a hydrophobic surface (region) and a region (nanowire region) surrounded by the hydrophobic surface, wherein the nanowire region includes hydrophilic nanowires; introducing an aqueous solution onto the surface of the substrate; and isolating the aqueous solution into the nanowire region.

## Description

### TECHNICAL FIELD

The present disclosure relates to a method for forming a microdroplet array.

### BACKGROUND ART

Various microdroplet arrays have previously been developed. With microdroplets arranged in a two-dimensional array, better operability, a faster reaction in the microdroplets, a smaller amount of a reagent used, etc. can be achieved than with previous techniques such as an automatic pipettor and a fluid device. Microdroplet arrays in which a sample subjected to so-called "terminal dilution" or "limiting dilution" is dispersed in a large number of microdroplets are used for high-sensitivity, high-precision, and high-speed experiments, analyses, diagnoses, etc.

The range of applications of the two-dimensional microdroplet arrays extends across various fields including biochemistry, chemistry, medical sciences, etc. For example, the microdroplet arrays have been practically used for droplet digital PCR (ddPCR) (NPL 1, digital ELISA (NPL 2), etc. Quantification can be made without using a calibration curve.

In one example of the microdroplet array, a hydrophobic surface and a hydrophilic surface are prepared on a flat surface, and droplets are formed on the hydrophilic surface with the aid of surface tension. The volume of the droplets is limited by their bottom area and the surface tension. Therefore, the available size is limited, and, in particular, nanosized droplets cannot be obtained. The surface tension is determined by the physicochemical properties of the hydrophilic surface. Therefore, the droplets are influenced by the production conditions, the shelf time after production, exposure to the environment, etc. Thus, it is costly or difficult to control the chemical properties of the hydrophilic surface immediately before use.

In another example of the microdroplet array, a large number of microwells are formed on the surface of a substrate. The microwells are generally formed using a microprocessing technique including lithography and etching. The size of the microwells or its precision depends on the level of the microprocessing technique. In particular, nano-processing is difficult to perform. Even when the nano-processing is possible, the cost of the processing is high. To isolate a solution into the microwells, first, the solution is introduced onto the surface of the substrate on which the microwells are arranged, and then the solution present outside the microwells is discharged to the outside using, for example, oil. In this case, if the microwells are small, e.g., their size is about 1 µm or less, the solution cannot easily enter the microwells. Moreover, the solution in the microwells tends to be discharged to the outside together with the oil and is unlikely to remain in the microwells.

Moreover, problems have been pointed out in other applications. In digital ELISA, a primary antibody is generally immobilized on a support such as the bottoms of wells or beads, and the efficiency of the reaction between the primary antibody and a target substance is low. Moreover, it has been said that the reaction efficiency of a spherical target substance such as extracellular vesicles is low because their orientation or position with respect to the primary antibody is not constant. Therefore, the target substance tends to be lost in a washing step.

Generally, in ddPCR, target molecules, a primer, and a polymerase are first mixed, and the mixed solution is diluted and then introduced into the system to form droplets. Therefore, only a part of the prepared solution is used. Thus, when the concentration of thin target molecules is low such as in single molecule measurement, it is necessary to perform pre-amplification.

### CITATION LIST

### NON PATENT LITERATURE

NPL 1: "dPCR: A Technology Review," Phenix-Lan Quan et al., Sensors (Basel). 2018 Apr; 18(4): 1271
NPL 2: "Mobile platform for rapid sub-picogram-per-milliliter," multiplexed, digital droplet detection of proteins, Venkata Yelleswarapu et al., PNAS, 116 (10) 4489-4495

### Summary of Invention

One embodiment of the present disclosure provides a method for forming droplets. In some embodiments, the method includes: providing a device, a flow channel, and/or a substrate having a surface including a hydrophobic surface (region) and regions (nanowire regions) surrounded by the hydrophobic surface, wherein each of the nanowire regions includes hydrophilic nanowires; introducing an aqueous solution onto the surface of the substrate; and isolating the aqueous solution into the nanowire regions.

In this manner, for example, the volume of the microdroplets can be controlled over a wide size range. In particular, nanosized droplets can be formed.

In some embodiment of the method, a digital assay can be performed. For example, target molecules can be captured on the nanowires. Therefore, the target molecules captured on the nanowires are unlikely to be lost in a washing step. For example, in digital ELISA, first, a primary antibody may be captured on the nanowires, and then an antigen and a secondary antibody may be introduced. Alternatively, an antigen may be captured on the nanowires, and then a detection antibody may be introduced. For example, in ddPCR, a solution containing target molecules may be introduced and concentrated by drying treatment, and then the target molecules may be captured on the nanowires. Moreover, for example, in ddPCR, target molecules may be captured on the nanowires, and then a primer and a polymerase may be introduced. In this manner, smaller droplets, e.g., nanosized droplets, can be used to reduce the volume of the droplets. The concentrations of the nanowires and the captured substances are thereby increased, and the capture efficiency is increased, so that measurement on low-concentration target molecules can be performed with high precision.

Additional aspects and advantages of the present disclosure will become readily apparent to those skilled in the art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF DRAWINGS

[Fig. 1] A flowchart showing a method for forming droplets in an embodiment.
[Fig. 2] Cross-sectional views of a substrate that illustrate a method for forming the substrate used to form droplets in an embodiment.
[Fig. 3] A layout of a mask used to develop spot nanowire regions in an Example.
[Fig. 4] SEM images of top views of spot nanowires in two Examples.
[Fig. 5] SEM images of top views of spot nanowires in two Examples.
[Fig. 6] SEM images of side views of spot nanowires in the two Examples.
[Fig. 7] A three-dimensional fluorescence microscope image used to measure the shapes of droplets in an Example.
[Fig. 8] An exploded perspective view of a flow channel device in an Example.
[Fig. 9] Cross-sectional views of a substrate that illustrate a process for forming droplets in an embodiment.
[Fig. 10] Cross-sectional views of a substrate that illustrate a process for capturing a target substance in an aqueous solution in an embodiment.
[Fig. 11] A fluorescence image (A) and a bright-filed image (B) of spot nanowire spaces in an Example.
[Fig. 12] Graphs showing the relation between the intensity of fluorescence measured in spot nanowire spaces and the frequency in three Examples.
[Fig. 13] Schematic illustrations showing the steps of preparing a device having spot nanowire spaces, capturing EVs on the nanowires, and detecting membrane proteins of the captured EVs in some embodiments.
[Fig. 14] The distributions of fluorescence intensities of mCherry and TokyoGreen in a negative control and captured fluorescently labeled EVs in an Example.
[Fig. 15] Expression frequencies of EVCLDN3/CD9 and TACSTD2/CD9 derived from urine of HGSOC patients in an Example.

### DESCRIPTION OF EMBODIMENTS

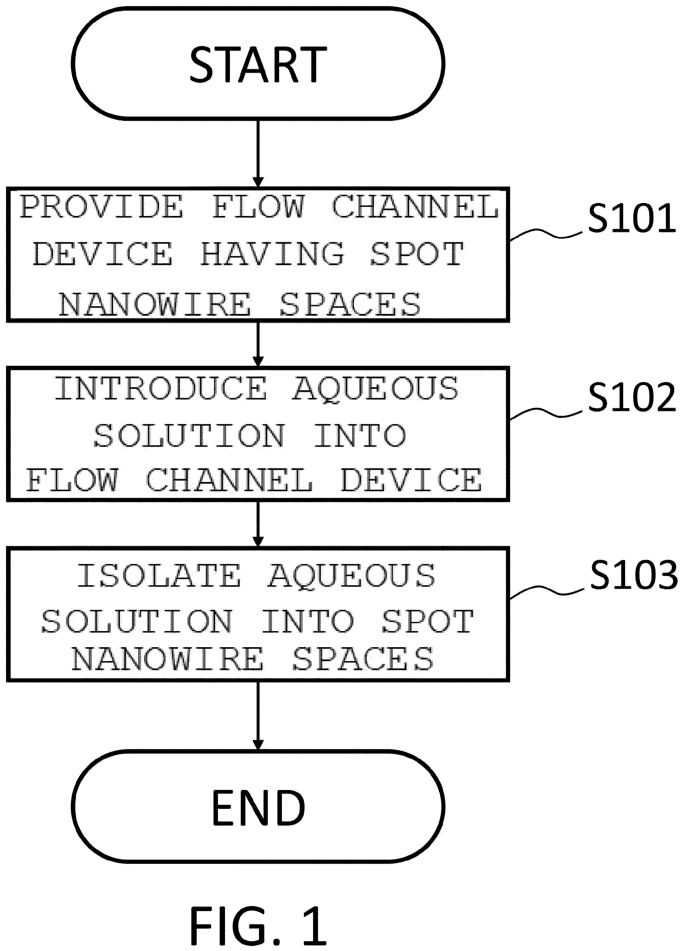

Fig. 1 shows a flowchart of a method for processing an aqueous solution or a method for forming droplets in an embodiment of the present disclosure. First, a flow channel device (or a substrate) having nano-spot spaces is provided (S101). An aqueous solution is introduced into the flow channel device (S102). The introduced aqueous solution is isolated into the nano-spot spaces (droplets are formed) (S103).

In some embodiments, a device or method for isolating a target substance contained in an aqueous solution or for isolating and/or capturing the target substance into nano-spot spaces is provided. In some embodiments, a device or method for measuring the target substance isolated or captured in the nano-spot spaces is provided.

### <Aqueous solution>

In the present disclosure, the aqueous solution introduced into the flow channel may be a body fluid. The "body fluid" means a body fluid obtained from a subject or a sample derived from the body fluid. The body fluid may be, but is not limited to, blood, serum, plasma, or lymph, may be tissue fluid such as interstitial fluid (intercellular fluid) or intercellular fluid, or may be body cavity fluid, serous cavity fluid, pleural fluid, ascites fluid, pericardial fluid, cerebrospinal fluid (spinal fluid), joint fluid (synovial fluid), or humor aqueous (aqueous humor). The body fluid may be a digestive fluid such as saliva, gastric juice, bile, pancreatic juice, or intestinal fluid or may be sweat, tears, snivel, urine, semen, vaginal fluid, amniotic fluid, or milk. The body fluid may be an animal body fluid or a human body fluid.

The "body fluid" may be derived from a subject of a body fluid test. The test subject may be an animal. The test subject may be a reptile, a mammal, or an amphibian. The mammal may be a dog, a cat, a cow, a horse, a sheep, a pig, a hamster, a mouse, a squirrel, or a primate such as a monkey, a gorilla, a chimpanzee, a bonobo, or a human. **In** particular, the test subject may be a human.

The aqueous solution may be, for example, a body fluid or a liquid derived from a body fluid (such as a diluted solution or a treated solution). The aqueous solution may be a liquid other than a body fluid (derived from a non-body fluid), may be an artificially prepared liquid, or may be a mixture of a body fluid or an aqueous solution derived from a body fluid and an aqueous solution derived from a non-body fluid. The aqueous solution may be an aqueous solution used for sample measurement or may be a solution used for measurement for calibration. The aqueous solution may be, for example, a standard solution or a calibration solution. The sample used for the measurement may be a specimen. The aqueous solution may contain a physiological buffer solution, such as phosphate buffered saline (PBS) or an N-tris(hydroxymethyl)methyl-2-aminoethanesulfonic acid buffer solution (TES), containing the substance to be collected.

**In** some embodiments, the aqueous solution may be a solution for performing biological, chemical, biochemical, physical, or optical measurement, other measurement, or a combination thereof. For example, the aqueous solution may be a solution for performing PCR (polymerase chain reaction).

### <Target substance>

In some embodiments, the aqueous solution or aqueous liquid (these terms are used herein interchangeably) may contain a target substance. The "target substance" used herein is generally a substance captured in droplets formed (nanowire spaces) and used or usable for measurement, detection, observation, quantification, calibration, etc. In some embodiments, the target substance may be an inorganic substance or an organic substance. In some embodiments, the target substance may be biomolecules.

The biomolecules may be cell organelles or vesicles. The vesicles may be, but are not limited to, vacuoles, lysosomes, transport vesicles, secretory, gas vesicles, extracellular matrix vesicles, extracellular vesicles, etc. or may include a plurality of types. The extracellular vesicles (EVs) may be, but are not limited to, exosomes, exotomes, shedding microvesicles, microvesicles, membrane particles, a plasma membrane, poptotic vesicles, etc. The vesicles may contain a nucleic acid.

The biomolecules may be, but are not limited to, cells or may include cells. The cells may be red blood cells, white blood cells, immune cells, etc. The biomolecules may be viruses, bacteria, etc. Non-limiting examples of the viruses include influenza viruses, coronaviruses, and dengue viruses.

The biomolecules may be nucleic acid, i.e., deoxyribonucleic acid (DNA) and/or ribonucleic acid (RNA). The RNA may be messenger RNA (mRNA), transfer RNA (tRNA), ribosomal RNA (rRNA), microRNA (miRNA), etc. The DNA/RNA may be, for example, cell-free DNA/RNA (cfDNA/RNA) or intracellular (e.g., intraorganellar) DNA/RNA. The DNA/RNA may be obtained by liquid biopsy. The nucleic acid may be subjected to modification such as methylation. The biomolecules may be a protein.

In some embodiments, the target substance may be biomolecules contained in a specific type of biomolecules (e.g., cells or vesicles).

### <Substrate>

A "substrate" as used herein is generally any substrate or a material surface formed on a substrate on which film processing is performed during a production process. For example, the surface of the substrate to be subjected to the processing contains a material such as silicon (Si), silicon oxide (SiO₂), silicon on insulator (SOI), germanium (Ge), gallium arsenide (GaAs), glass, or sapphire. The surface of the substrate contains a semiconductor material or a material generally used for a semiconductor process. The surface of the substrate contains another material such as a metal, a metal oxide, a metal alloy, or another electrically conductive material according to the intended application. The surface of the substrate may contain a polymeric material. At an atomic or micrometer level, an oxide, a lipid component, etc. may be present on the surface of the substrate. Examples of the substrate include, but are not limited to, semiconductor wafers such as silicon wafers, glass wafers, and polymer films. The substrate may be subjected to a pretreatment process to polish, etch, reduce, oxidize, hydroxylate, anneal, clean, and/or bake the substrate surface. The surface of the substrate contains any of the above materials as a basic component and may include a surface layer or contain impurities when the substrate is handled, for example, in air or in a clean room environment.

The surface of the substrate may be substantially flat. The surface of the substrate may include a flat surface. The substrate may be bonded to a member defining the flow channel such that the flow channel formed includes the substrate surface on its inner side. A flow channel space may be substantially fluid-tightly sealed or may have an introduction port (inlet) or a discharge port (outlet). The inlet and the outlet may be provided separately, or one opening or the same opening may be used for both the inlet and the outlet. In some embodiments, at least part of the surface of the substrate may be a curved surface.

### <Hydrophobic surface>

In some embodiments, part or all of the surface of the substrate may be hydrophobic or may be formed of a hydrophobic material or coated with a hydrophobic film. The "hydrophobic film" used herein is a film formed of a material having hydrophobicity (a hydrophobic material, the same applies to the following) or having a hydrophobic material on its surface. The hydrophobic film may contain a fluorine compound or a fluorocarbon resin. The hydrophobic film may be a so-called fluorocarbon resin film.

The hydrophobic material may be selected from the group consisting of fluorinated polymers, perfluorocarbon polymers, silicon polymers, and mixtures thereof. Examples of the hydrophobic material include amorphous fluoropolymers (commercial examples thereof include: the CYTOP (registered trademark) series manufactured by AGC Chemicals and having one of the following terminal functional groups. The hydrophobic material has, for example, one of the following terminal functional groups: type A: -COON, type M: -CONH-Si(OR), and type S: -CF₃); polytetrafluoroethylene (commercial examples thereof include TEFLON (registered trademark) manufactured by Chemours); parylene; fluorinated hydrocarbons; fluoroacrylic copolymers (commercial examples thereof include FLUOROPEL (registered trademark) manufactured by Cytonix); fluorosilanes (for example, such as trichloro(1H,1H,2H,2H-perfluorooctyl)silane (PFOTS) and perfluorodecyltrichlorosilane (FDTS)); plasma-deposited fluorocarbons; polydimethylsiloxane; other siloxanes; hydrophobic hydrocarbons such as 1-heptadecyne; and mixtures thereof.

The hydrophobic material may be applied to at least part of the substrate surface or the entire substrate surface. The hydrophobic material may be applied to the surface of the substrate by a dry or wet process. For example, the hydrophobic material in a solution state may be dropped onto the surface of the substrate to coat the surface by spin coating. Then the hydrophobic material may be dried, heat-treated, or baked.

### <Nanowire regions>

The "nanowire regions" as used herein are each a surface region on the substrate surface from which nanowires grow, on which nanowires are disposed, or on which ends or part of nanowires are held. When the nanowires extend outward from surface regions of a seed material in three-dimensional directions, the nanowire regions may be defined by outlines formed by projecting the forward ends of the nanowires onto the substrate surface from above in the perpendicular direction. One nanowire region may be defined by a circle, a closed curve other than a circle, a polygon, a closed zigzag line, or a combination thereof.

Each nanowire region is surrounded by the hydrophobic surface. As described later, at least the surface of each nanowire has hydrophilicity.

A plurality of nanowire regions may be disposed on the surface of the substrate. The plurality of nanoregions or the plurality of nanowire spaces on the surface of the substrate may be arranged regularly, geometrically, or in an array, may be arranged randomly, or may be arranged in a combination of any of these forms.

### <Formation of nanowire regions>

In some embodiments, the hydrophobic film formed on the surface of the substrate is patterned to form openings. In some embodiments, the hydrophobic film may be pattered using photolithography. A resist is applied to the surface of a plastic film using a spin coater or by spraying, prebaked, exposed to light, and developed. The resist is thereby patterned. The hydrophobic film is exposed at portions from which the resist has been removed. After the development, treatment such as rinsing and post-baking may be performed.

In some embodiments, the patterning may include etching using a hard mask (e.g., a metal mask).

In some embodiments, the exposed surface of the hydrophobic film is subjected to etching treatment. In this manner, the hydrophobic film is patterned according to the pattern of the resist. No particular limitation is imposed on the etching of the hydrophobic film, but oxygen etching, for example, may be used. In this manner, the surface of the substrate is exposed.

The size, diameter, or radius of the apertures (nanowire regions) may be less than or equal to any of the following values: 100 µm, 70 µm, 50 µm, 30 µm, 20 µm, 10 µm, 7 µm, 5 µm, 3 µm, 2 µm, 1 µm, 700 nm, 500 nm, 300 nm, 200 nm, 100 nm, 70 nm, 50 nm, 30 nm, 20 nm, 10 nm, etc. The limit of the size of the apertures (nanowire regions) is generally determined by the precision of lithography. For example, UV (ultraviolet) lithography, EUV (extreme ultraviolet) lithography, EB (electron beam) lithography, etc. may be used.

The number of nanowire spaces the substrate can have in one space may be 1,000, 10,000, 20,000, 30,000, 50,000, 70,000, 100,000, 200,000, 300,000, 500,000, 700,000, 1,000,000, 2,000,000, 3,000,000, 5,000,000, 7,000,000, 10,000,000, 20,000,000, 30,000,000, 50,000,000, 70,000,000, 100,000,000, 200,000,000, 300,000,000, 1,000,000,000, 2,000,000,000, 3,000,000,000, 5,000,000,000, 7,000,000,000, 10,000,000,000, etc., or the substrate can have a larger number of nanowire spaces.

The area of the region in which the plurality of nanowire spaces are arranged in one space may be less than or equal to any of the following values: 100 cm², 70 cm², 50 cm², 30 cm², 20 cm², 10 cm², 9 cm², 8 cm², 7 cm², 6 cm², 5 cm², 4 cm², 3 cm², 2 cm², 1 cm², 0.9 cm², 0.8 cm², 0.7 cm², 0.6 cm², 0.5 cm², 0.4 cm², 0.3 cm², 0.2 cm², 0.1 cm², etc.

With a conventional method for forming microdroplets, e.g., microwells, it is difficult to "cut" droplets with a size of about 1 µm or smaller. Therefore, it is difficult to densely arrange the microdroplets. However, in the embodiment of the present disclosure, the nanowire spaces can be defined by their bottom surfaces, i.e., the nanowire regions, and can be densely arranged. For example, when UV lithography is used, nanowire regions with a size of the order of µm to several hundreds of nm can be formed. When an EB drawing device is used, smaller nanowire regions can be formed. Therefore, theoretically, a microdroplet array with a density suitable for the structural limit of an optical microscope or an image sensor can be produced.

### <Application of seed material>

In some embodiments, the nanowires are formed in aperture portions or nanowire regions provided in the hydrophobic region on the surface of the substrate. For example, particles or a catalyst used to grow the nanowires may be applied to the surface of a seed material, and the resulting surface may be used as starting points for the growth of the nanowires. **In** some embodiments, the material may be a material that can serve as a catalyst or starting points for the growth of the nanowires.

In some embodiments, the seed material for the growth of the nanowires may be applied to the exposed surface of the substrate. The seed material may be a material that can serve as a catalyst or starting points for the growth of the nanowires. The seed material may be applied to the entire substrate, i.e., the surface of the resist. A film containing the seed material or composed substantially of a target material may be formed.

### <Removal of photoresist>

In some embodiments, the photoresist may be removed after the application of the seed material. In this case, the target material applied to the surface of the photoresist may also be removed together with the photoresist. This process may be referred to as lift-off. When the lift-off is performed, the hydrophobic film and the patterned seed material may remain on the surface of the substrate.

### <Treatment of seed material>

In some embodiments, the applied target material may be subjected to treatment. The treatment may be performed on the surface of the target material. The treatment may be heating. The heating may include, for example, heating the substrate in a heating furnace or irradiating the target material or the substrate with a laser beam. The treatment may be oxidation of at least the surface of the target material or part or all of the volume of the target material. Examples of the oxidation include heating in an oxidizing atmosphere and plasma treatment. In some embodiments, the seed material may not be pre-treated before the growth of the nanowires. For example, when a hydrophobic film sensitive to temperature is used, heating treatment may be avoided.

### <Nanowires>

When the radius or diameter of the "nanowires" used herein, their characteristic size, their diameter, etc. are not specified, the nanowires are structural bodies in which their maximum diameter, minimum diameter, average diameter, and other characteristic sizes in a cross section are at a nanometer (nm) level, a sub-nanometer level, a 10 nanometer level, a 100 nanometer level, or a sub-micrometer level. The length of a "nanowire" is its size in the longitudinal direction and may be at a nanometer level, a 10 nanometer level, a 100 nanometer level, or a sub-micrometer level. The "nanowire" means a rod-shaped or wire-shaped structural body having a size such as a diameter or a cross-sectional shape of the order of nanometers (having, for example, a diameter of, but not limited to, 1 nanometer to several hundreds of nanometers). In some modes, the length of the nanowires described herein is about 0.1 nanometers to about 500 nanometers, about 1 nanometer to about 250 nanometers, about 1 nanometer to about 100 nanometers, or about 5 nanometers to about 50 nanometers.

The length of the nanowires may be, but is not limited to, for example, more than or equal to any of the following values: 500 nm, 1 µm, 1.5 µm, 2 µm, 3 µm, 4 µm, 5 µm, 6 µm, 7 µm, 8 µm, 9 µm, 10 µm, 11 µm, 12 µm, 13 µm, 14 µm, 15 µm, 17 µm, 20 µm, etc. The length of the nanowires may be, but is not limited to, for example, less than or equal to any of the following values: 1 µm, 1.5 µm, 2 µm, 3 µm, 4 µm, 5 µm, 6 µm, 7 µm, 8 µm, 9 µm, 10 µm, 11 µm, 12 µm, 13 µm, 14 µm, 15 µm, 17 µm, 20 µm, 50 µm, 100 µm, 200 µm, etc.

The diameter of the nanowires (or their size in the thickness direction) may be, but is not limited to, for example, more than or equal to any of the following values: 5 nm, 10 nm, 15 nm, 20 nm, 25 nm, 30 nm, 40 nm, 50 nm, 60 nm, 70 nm, 80 nm, 90 nm, 100 nm, 150 nm, 200 nm, 250 nm, 300 nm, 400 nm, 500 nm, etc. The diameter of the nanowires (or their size in the thickness direction) may be, but is not limited to, for example, less than or equal to any of the following values: 10 nm, 15 nm, 20 nm, 25 nm, 30 nm, 40 nm, 50 nm, 60 nm, 70 nm, 80 nm, 90 nm, 100 nm, 150 nm, 200 nm, 250 nm, 300 nm, 400 nm, 500 nm, 1 µm, etc.

The cross section of each nanowire may have a substantially circular, elliptical, regular polygonal, or polygonal shape or any other shape. The outer shape of each nanowire may have a substantially cylindrical, elliptic cylindrical, or polygonal cylindrical shape or any other shape. The nanowires may be hollow bodies or bodies with cavities or may be structural bodies substantially filled with a material.

In some embodiments, the nanowires may be formed directly on the substrate with no catalyst layer interposed therebetween or with no catalyst layer formed. In some embodiments, the surface (inner wall) of the substrate or the surface of a catalyst layer on which the nanowires are to be formed or grown may be subjected to surface treatment such as activation treatment, hydrophilization treatment, heat treatment, or hydrothermal treatment. The surface treatment may be, for example, plasma treatment, particle (ion, radical, neutral atom, etc.) beam irradiation, irradiation with light (electromagnetic wave) such as UV or EUV, electron beam irradiation, or mechanical treatment such as polishing. The surface treatment may be, for example, treatment for increasing the presence of oxygen that is to be bonded to a metal to form a Lewis acid. In some embodiments, performing the surface treatment may include performing a plurality of types of surface treatment. In some embodiments, two, two or more, or a plurality of types of surface treatment may be performed simultaneously, sequentially, or in combination.

The nanowires may be grown using any of the following methods: physical vapor deposition methods such as pulse laser deposition and a VLS (Vapor-Liquid-Solid) method, a CVD (Chemical-Vapor-Deposition) method, an arc discharge method, a laser evaporation method, a metal-organic vapor phase selective growth method, a hydrothermal synthesis method, a reactive ion etching method, a firing method, a melting method, a sputtering method, etc.

The material of the nanowires may be an inorganic material or an organic material. The nanowires may be formed of or contain a metal, a non-metal, a semiconductor, a mixture or alloy thereof, or an oxide or nitride thereof. The material of the nanowires may be or contain a polymeric material. The nanowires may be wires, whiskers, fibers, or mixtures or composites thereof.

Examples of the metal used as the material of the nanowires include, but are not limited to, typical metals (alkali metals: Li, Na, K, Rb, and Cs and alkaline-earth metals: Ca, Sr, Ba, and Ra), magnesium group elements: Be, Mg, Zn, Cd, and Hg, aluminum group elements: Al, Ga, and In, rare earth elements: Y, La, Ce, Pr, Nd, Sm, and Eu, tin group elements: Ti, Zr, Sn, Hf, Pb, and Th, iron group elements: Fe, Co, and Ni, earth-acid elements: V, Nb, and Ta, chromium group elements: Cr, Mo, W, and U, manganese groups elements: Mn and Re, noble metals (copper group and coinage metals): Cu, Ag, and Au, platinum group elements: Ru, Rh, Pd, Os, Ir, and Pt, natural radioactive elements: U and Th-based radioactive decay products: U, Th, Ra, Rn, and actinoids, transuranic elements: elements with atomic numbers larger than that of uranium such as Np, Pu, Am, Cm, Bk, Cf, Es, Fm, Md, and No, and alloys thereof. The nanowires may be formed of an oxide of any one of the above metals and alloys, an oxide of an alloy, or an oxide of a mixture of any of the above metals and alloys or may contain any of the above oxides.

The nanowires may be formed of one material or may be formed of a plurality of materials. The surfaces of the nanowires may be coated with a coating material.

The material of the nanowires or at least the surfaces of the nanowires (e.g., the coating material) may be, but is not limited to, for example, ZnO, SiO₂, Li₂O, MgO, Al₂O₃, CaO, TiO₂, Mn₂O₃, Fe₂O₃, CoO, NiO, CuO, Ga₂O₃, SrO, In₂O₃, SnO₂, Sm₂O₃, EuO, etc.

The nanowires may be electrically charged. The nanowires may have opposite charges to the material to be collected or extracted. In one non-limiting example, the charged nanowires can attract or adsorb charged biomolecules such as extracellular vesicles, nucleic acids, etc.

### <Seed material>

In some embodiments, the nanowires may be grown via a catalyst layer, a bonding layer, growth nucleus, or a seed material (layer) (these terms are used herein interchangeably and may be referred to simply as a "seed material"). The "layer" may be a thin film. The "layer" may be a continuous film. The "layer" may be discontinuous. The "layer" may be a continuous film, and the film may have holes. The "layer" may be a plurality of separate thin films. The "layer" may be an island or may include an island. The "layer" may be particles or may include particles.

The catalyst layer, the bonding layer, the growth nucleus, or the seed material layer may be formed of a metal, an alloy, a non-metal, a semiconductor, an oxide or nitride thereof, or a mixture thereof. Examples of the metal include, but are not limited to, typical metals (alkali metals: Li, Na, K, Rb, and Cs and alkaline-earth metals: Ca, Sr, Ba, and Ra), magnesium group elements: Be, Mg, Zn, Cd, and Hg, aluminum group elements: Al, Ga, and In, rare earth elements: Y, La, Ce, Pr, Nd, Sm, and Eu, tin group elements: Ti, Zr, Sn, Hf, Pb, and Th, iron group elements: Fe, Co, and Ni, earth-acid elements: V, Nb, and Ta, chromium group elements: Cr, Mo, W, and U, manganese groups elements: Mn and Re, noble metals (copper group and coinage metals): Cu, Ag, and Au, platinum group elements: Ru, Rh, Pd, Os, Ir, and Pt, natural radioactive elements: U and Th-based radioactive decay products: U, Th, Ra, Rn, and actinoids, and transuranic elements: elements with atomic numbers larger than that of uranium such as Np, Pu, Am, Cm, Bk, Cf, Es, Fm, Md, and No. The oxide may be an oxide of one of these metals and alloys.

The particles or the seed layer for forming the nanowires may be formed of, for example, Cr, ZnO, or CrO. Nanowires formed using ZnO can be grown on the surface of the seed material using a hydrothermal synthesis method. In one example, first, ZnO is applied to the surface of the substrate (the surface of the target material). Next, the substrate is immersed in a precursor solution prepared by dissolving zinc nitrate hexahydrate (Zn(NO₃)₂·6H₂O) and hexamethylenetetramine (C₆H₁₂N₄) in deionized water. In this manner, ZnO nanowires can be grown on the surface of the seed material.

The seed material for the nanowires may be formed of a material different from the material of the nanowires. The seed material for the nanowires may be formed of substantially the same material as the material of the substrate. The seed material for the nanowires may have, for example, a surface having structural irregularities. The seed material for the nanowires may have, for example, a surface including portions with different chemical properties. On the (mottled) surface including portions with different mechanical, structural, or chemical properties, some portions may be more likely to serve as a seed material for the nanowires than the other portions.

In some embodiments, it is not always necessary to form the seed material. For example, the nanowires may be grown directly on the surface of the substrate or on the surface of another layer (other than the seed material). For example, irregularities may be formed by lithography and dry-wet etching etc. For example, the (mottled) surface including portions with different mechanical, structural, or chemical properties may be formed by irradiation with ions, neutral atoms, plasma, etc.

### <Spot nanowire spaces>

The nanowires are disposed in a direction perpendicular to or inclined with respect to the surface of the substrate, i.e., arranged three-dimensionally. In this manner, effective spaces for holding the aqueous solution can be defined. Herein, these spaces are interchangeably referred to as nanowire spaces, spot-shaped nanowire spaces, spot nanowire spaces, nano-spot spaces, spot spaces, and dot nanowire spaces. The nanowire spaces determine the volumes of droplets formed therein. Herein, the nanowires forming the spot nanowire spaces are interchangeably referred to as spot nanowires and dot nanowires.

The spacing between the spot nanowire regions and their bottom area can be determined by lithography. The three-dimensional sizes of the spot nanowire spaces such as their height and width can be controlled by changing the growth conditions of the nanowires. Examples of the growth conditions include the concentration of a growth solution and the growth time.

In some embodiments, the volume of each spot nanowire space may be defined using its bottom surface and the space in which the nanowires extend. The three-dimensional shape of the spot nanowire spaces may be approximated, for example, by a cylinder or a part of a sphere or an ellipsoid. The spaces in which the nanowires extend may be measured, for example, using electron microscopy (such as scanning electron microscopy (SEM)) or optical microscopy. In some embodiments, the volumes of the spot nanowire spaces may be determined actually or by simulations and defined as the volumes of the aqueous solution captured on the nanowires in the spot nanowire spaces. For example, the volumes of the droplets of the aqueous solution may be determined by taking images of the outlines of the droplets by optical microscopy. For example, a fluorescent substance is mixed into the aqueous solution, and fluorescent regions may be defined as the volumes of the spot nanowire spaces using fluorescence microscopy.

### <Method for forming nanowire spaces: example 1>

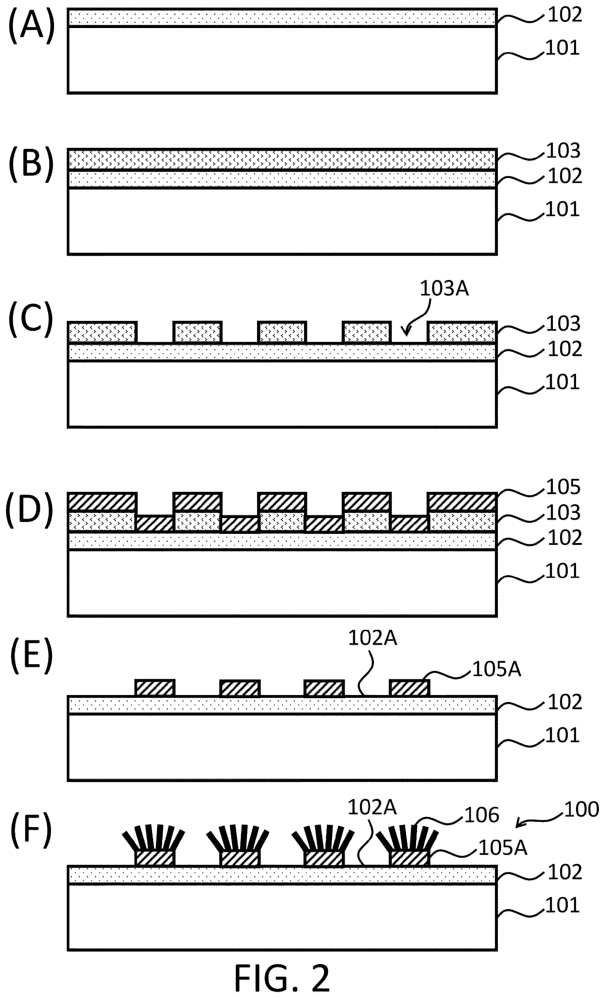

Fig. 2 shows a process for forming the spot nanowire spaces in one embodiment.

First, a substrate 101 is provided. Typically, the substrate 101 may be a semiconductor substrate such as a Si or SiO₂ substrate. Alternatively, the substrate 101 may be a polymeric substrate. A hydrophobic film 102 is formed on a surface of the substrate 101 (the substrate surface) (Fig. 2A). In Fig. 2, the surface of the substrate is coated with Cytop. However, the hydrophobic film 102 is not limited thereto, and another material and another method may be used to form the hydrophobic film 102.

A photoresist film 103 is formed on the hydrophobic film 102 (Fig. 2B).

The photoresist 103 is developed using a mask prepared such that portions in which nanowire regions are to be formed are apertures. In this manner, spots (apertures) 103A are formed by development, and the Cytop film (hydrophobic film) 102 is exposed from the spots 103A (Fig. 2C).

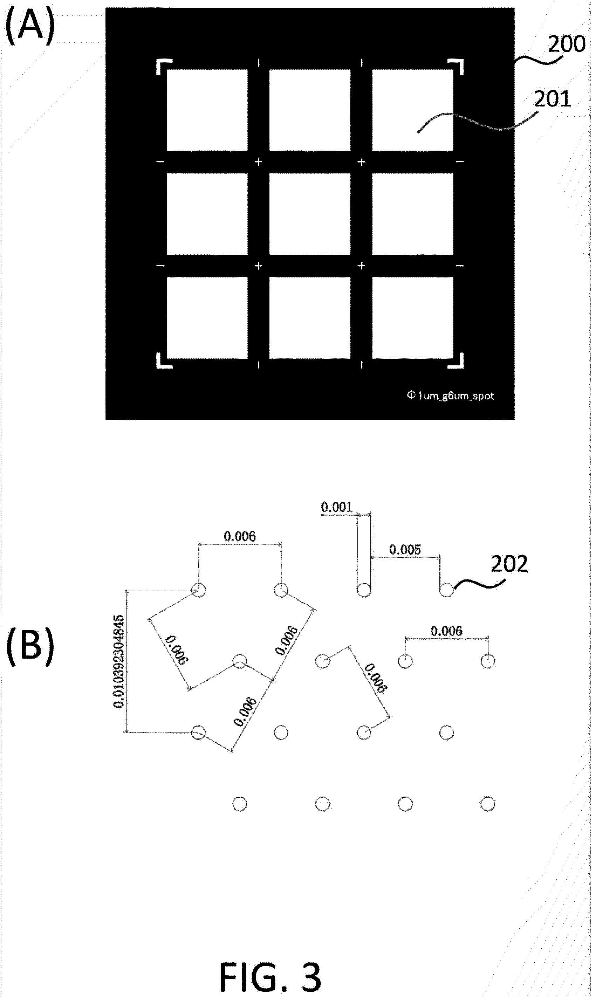

An example of the mask is shown in Fig. 3. The mask 200 used in this Example includes 9 segments (flow channel spaces), and each segment has an area of about 2 cm × 2 cm (Fig. 3A). Each segment 201 is surrounded and defined by an outline frame portion with no spots and corresponds to one flow channel device. In each segment, a large number of spots 202 are disposed at vertices of a large number of triangles with a side length of 6 µm (Fig. 3B). The distance between the spots in the figure is expressed in millimeters. In the mask 200, each segment 201 includes 1.28 × 10⁷ regions (spots 202). Specifically, the density is 3,200,000 spots/cm².

Next, a seed material 105 for the growth of nanowires is vapor-deposited by sputtering on the entire surface of the substrate (Fig. 2D). In Fig. 2, ZnO is vapor-deposited.

Then the photoresist 104 is removed (Fig. 2E). The photoresist 104 may be removed by so-called lift-off. In this manner, the seed material (ZnO) 105A remains on each spot 105A.

Next, a wet process is used to grow nanowires 106 on the surface of the seed material 105 formed on the spots 105A (Fig. 2F). The nanowires 106 are grown with the seed material (ZnO) 105 serving as starting points. No nanowires grow on the hydrophobic film 102. In this manner, the nanowires 106 are formed only in the spot regions 105A. Therefore, a large number of spot nanowire regions or spaces can be formed. Generally, the nanowires 106 have hydrophilicity and surrounded by a hydrophobic region 102A of the hydrophobic film 102.

### <Control of growth of nanowires: examples>

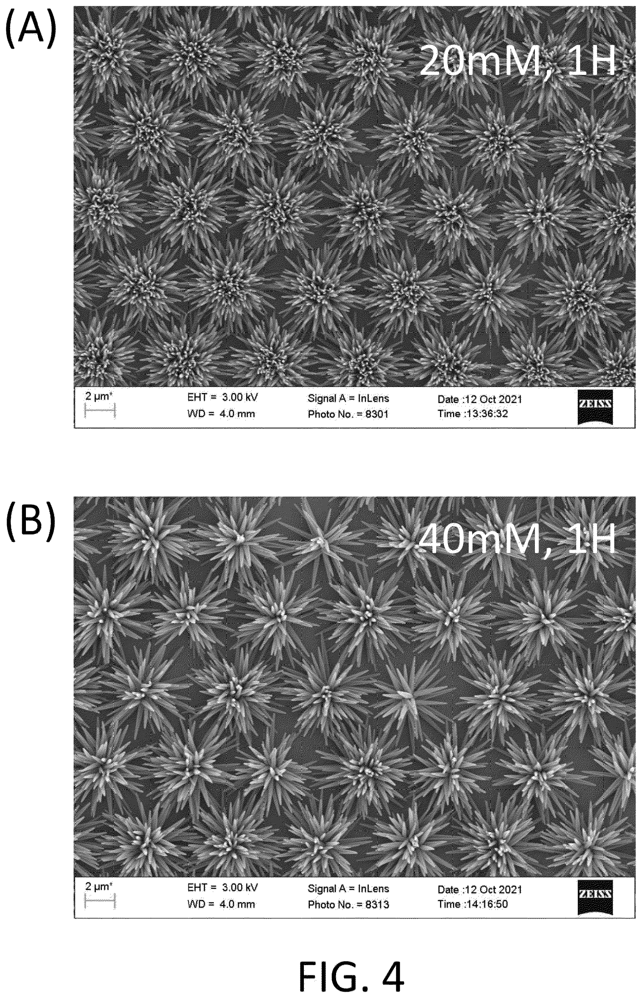

The influence of the concentration of the growth solution on the growth of nanowires was studied. Fig. 4 shows SEM images of nanowires grown on nanowire regions having the same plane shape using two growth solutions with different concentrations. Each growth solution contained zinc nitrate hexahydrate (Zn(NO₃)₂·6H₂O) and hexamethylenetetramine (C₆H₁₂N₄) at the same concentration. In these Examples, the concentration was 20 mM (Fig. 4A) and 40 mM (Fig. 4B). The growth time was 2 hours.

At 20 mM, narrow nanowires were formed (Fig. 4A). At 40 mM, nanowires thicker than those at 20 mM were formed (Fig. 4B). As described above, the concentrations of zinc nitrate hexahydrate (Zn(NO₃)₂·6H₂O) and hexamethylenetetramine (C₆H₁₂N₄) have an influence on the size (thickness, length, etc.) of the nanowires.

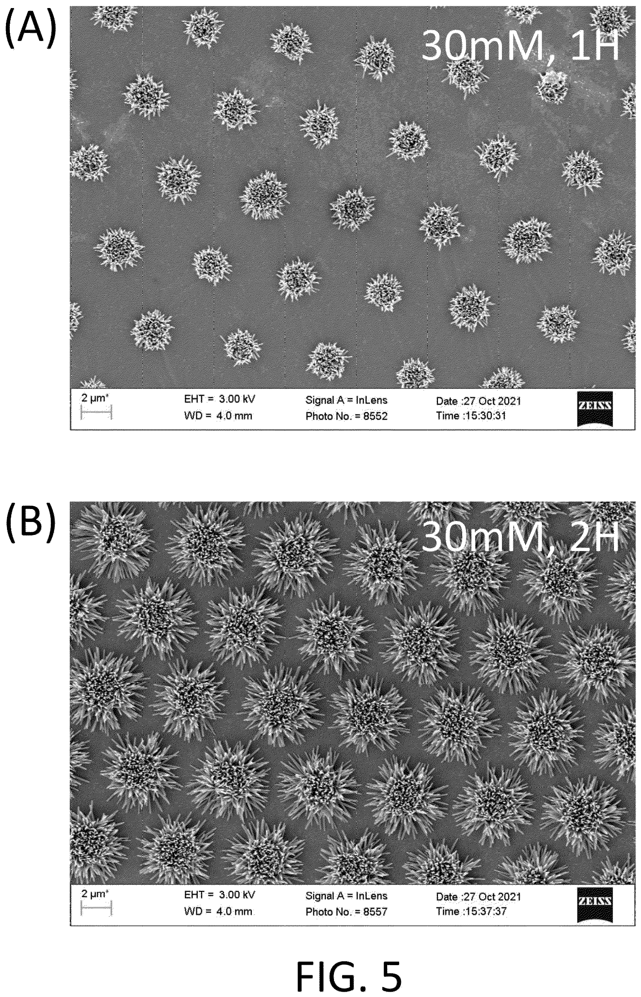

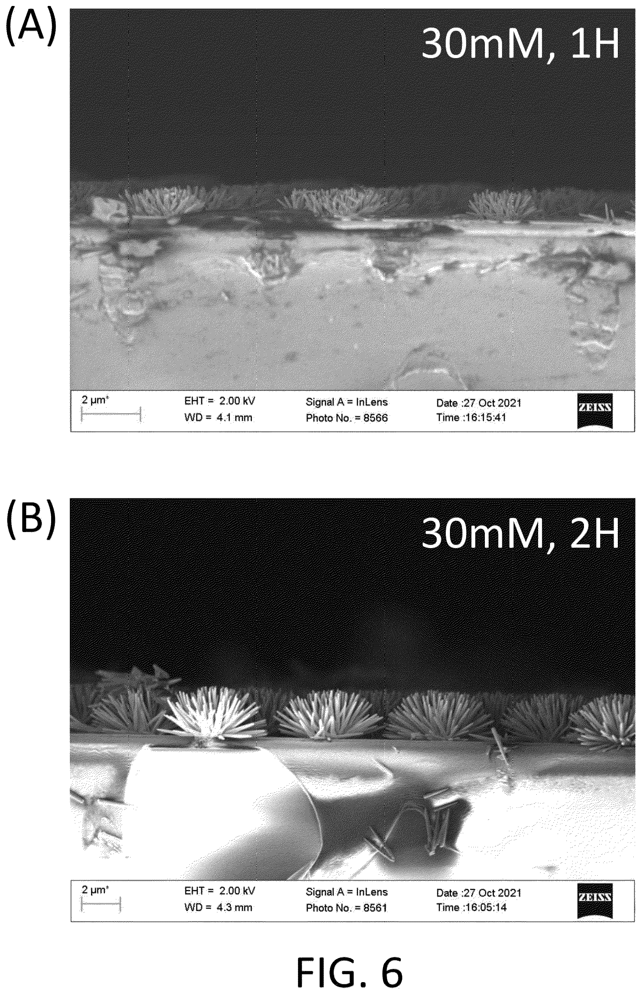

Figs. 5 and 6 show SEM images of nanowires grown in nanowire regions having the same plane shape at the same growth solution concentration (30 mM) for different times (1 hour and 2 hours). Fig. 5 shows a top image when the growth time was 1 hour (A) and a top image when the growth time was 2 hours (B). Fig. 6 shows a side image when the growth time was 1 hour (A) and a side image when the growth time was 2 hours (B).

When the growth time was 1 hour, about 150 to about 220 nanowires were formed per spot. As for the size of the nanowires, the diameter was 0.094±0.014 µm, and the length was 0.88±0.11 µm. When the growth time was 2 hours, the number of nanowires per spot was almost the same as that when the growth time was 1 hour. As for the size of the nanowires, both the diameter and the length were lager than those when the growth time was 1 hour.

When the growth time is 1 hour, the shape of the nanowire spaces can be approximated by a semi-sphere. As for the size of the semi-ellipsoid, the diameter was 3.66 µm, and the height was 3.22 µm. Therefore, the volume of the semi-ellipsoid was computed as V = 1/2 × 4/3 π × 1.83 µm × 1.83 µm × 3.22 µm = 22.6 fL.

The volume of the captured aqueous solution is more accurately computed by subtracting the volume of the nanowires from the volume of the space. For example, as for the size of the nanowires in the above Examples, the diameter was 0.094 µm, and the length was 0.882 µm, so that the volume was 0.0058 fL. Each nanowire space includes about 200 nanowires. Therefore, the volume of the nanowires in each nanowire space was computed as 1.2 fL. Thus, the volume of the aqueous solution captured in each nanowire space was computed as about 22.6 fL - 1.2 fL = 21.4 fL.

As described above, the volume of each of the femtoliter size nanowire spaces (which can be referred to also as reaction spaces or reaction fields) can be controlled by changing parameters of the nanowires such as their length.

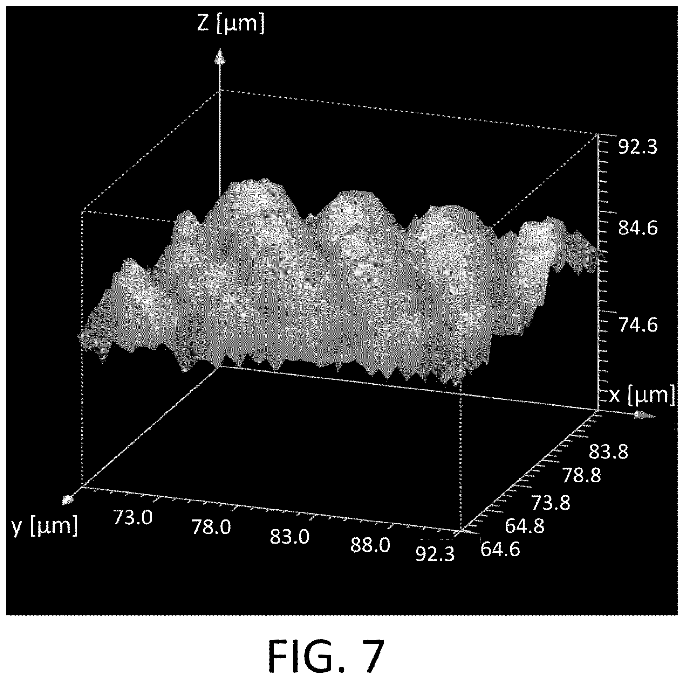

This complex aqueous solution was isolated in the same spot nanowire spaces as those described above. An image of the aqueous solution was taken using a fluorescence microscope (Leica, TCS SP5STED). The image is shown in Fig. 7. As can be seen from the image, the volume in which the aqueous solution is present, i.e., the shape of each droplet, can be approximated by a semi-sphere and is substantially the same as the volume defined by the shape of the nanowires.

### <Flow channel device>

In some embodiments, the surface of the substrate may be bonded to, for example, an upper substrate and may be disposed in a closed space. The surface of the substrate may be disposed inside a flow channel or a flow channel device. The fluid device may have a liquid inlet and a liquid outlet.

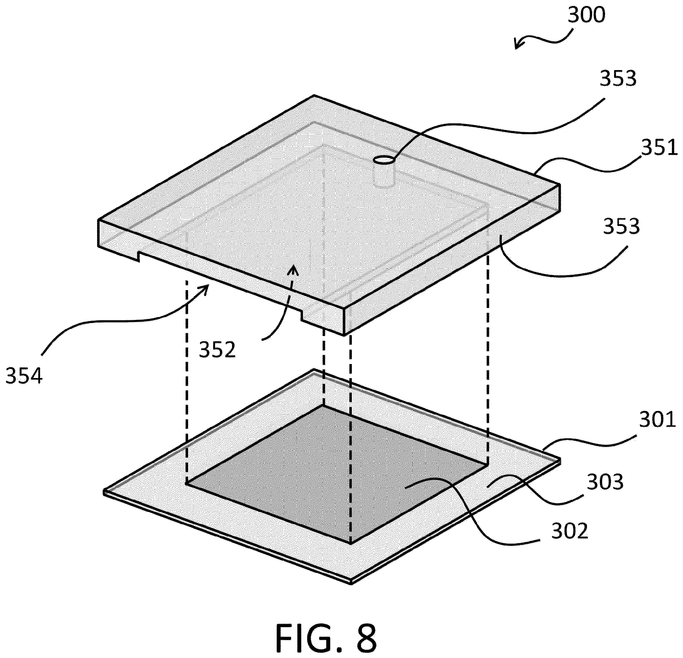

Fig. 8 shows a flow channel device 300 in one embodiment. The flow channel device 300 is produced by joining a first substrate 301 and a second substrate 351 together. The first substrate 301 has a hydrophobic surface (not shown) and a substrate surface 302 including a large number of nanowire regions (not shown) surrounded by the hydrophobic surface. The first substrate 301 has, in its edge portion, a bonding surface 303 surrounding the substrate surface 302. The second substrate 351 has a recessed portion (recessed space, flow channel-defining space) 352 disposed so as to face the substrate surface 302 of the first substrate 301. The second substrate 351 has, in its edge portion, a bonding surface 353 formed so as to be tightly bonded to the bonding surface 303 in the edge portion of the first substrate 301. The second substrate 351 has a solution inlet 353 formed so as to pass therethrough and extend from the recessed space 352 to the opposite surface (the upper surface of the flow channel device 300) and an outlet 354 formed as an apertures in one side of the flow channel device 300 that is opposite to the solution inlet 353 in a plane direction of the recessed portion space.

The device 300 formed by joining the first substrate 301 and the second substrate 351 together in the manner described above includes the recessed space (flow channel space) 352 defined by the substrate surface 302. The hydrophobic surface and a large number of nanowire regions surrounded by the hydrophobic surface are disposed on the substrate surface 302, i.e., disposed inside the recessed space (flow channel space) 352. A solution is introduced into the flow channel space 352 through the inlet 353. The aqueous solution present in spaces other than the nanowire spaces within the flow channel space 352 can be discharged through the outlet 354 by, for example, introducing air or oil through the inlet 353 or bringing a water-absorbing material into contact with the outlet 354. In this manner, the aqueous solution can be isolated in the nanowire spaces.

No particular limitation is imposed on the size and dimensions of the flow channel device. In one Example, the flow channel device may be designed as follows. The first substrate may be a SiO₂ substrate, and the second substrate may be a COP (cycloolefin polymer) substrate. The surface of the substrate corresponding to the flow channel space may have a size of 2 cm × 2 cm, as in Fig. 3. For example, 100 million or 20 million spots may be formed on the surface of the substrate. 1.28 × 10⁷ spots may be formed, as in Fig. 3. The height of the flow channel space may be 50 µm to 10 µm. As for the size of the inlet 353, the inlet 353 may be circular and have a diameter of 1 mm or 1.5 mm. The width of the outlet 354 in a plane direction of the substrate may be 2 cm, and its height may be 50 µm to 10 µm.

In some embodiments, the cover (second substrate) may have a plurality of inner spaces and a plurality of frames (not shown) surrounding the inner spaces. The cover having the plurality of inner spaces may be brought into close contact with a plurality of target material pattern portions and/or fine structures, and then the joined body including the substrate and the cover may be cut or separated according to the inner spaces.

In some embodiments, the cover (second substrate) may be transparent. In this case, light can be introduced or extracted, and this allows optical or fluorescent observation of the droplets.

In some embodiments, at least the surface of the cover (second substrate) on the flow channel space side may be formed of a hydrophobic material. In this case, for example, when the introduced aqueous solution is discharged, the aqueous solution can be prevented from remaining as droplets on the surface of the cover, or the amount of the remaining aqueous solution can be reduced. The hydrophobic material may be a polymeric material. For example, the material may be a cycloolefin polymer (COP).

The "flow channel" used in the present disclosure generally means a space for containing a liquid. The term "flow channel" can be used interchangeably with terms such as a fluid device, a fluid chamber, and a fluid container. The flow channel may have an inlet (introduction port) for introducing a fluid thereto and/or an outlet (discharge port) for discharging the fluid. The fluid may flow through the flow channel, or the fluid introduced into the flow channel may be substantially at rest.

In some embodiments, a chaotic mixer (chaotic mixing device) may be disposed in the flow channel, or a structure that causes the fluid flowing through the flow channel to form a nonlinear flow and/or a three-dimensional flow may be disposed in the flow channel. Such a structure may, for example, cause the flow channel to have a step or to change its cross section or may cause the direction of the flow channel to be changed.

A chaotic mixer having herringbone-like irregularities may be disposed on the inner wall of the flow channel (the surface of the substrate or the surface of the cover). In this case, the nonlinear flow of the fluid can be facilitated. This allows, for example, a larger amount of the target substance in the solution to be introduced into fine structures (e.g., the nanowires) disposed on a plurality of inner surfaces or curved inner surfaces.

The fluid device may detect, measure, analyze, modify, process, and/or capture a substance contained in the fluid introduced into the flow channel. The fluid device may include the nanowires. The fluid device may use the nanowires to capture a substance contained in the fluid. For example, the fluid device may capture biomolecules in a body fluid with the nanowires disposed in the flow channel.

### <Isolation of droplets>

In some embodiments, the aqueous solution may be introduced onto the surface of the substrate, and then the aqueous solution may be removed from the hydrophobic surface. In this manner, the aqueous solution can be isolated in the spot nanowire spaces.

### <Isolation using water-absorbing material>

In some embodiments, a water-absorbing material may be used to absorb the aqueous solution on the hydrophobic surface to thereby remove the aqueous solution from the hydrophobic surface. For example, the aqueous solution can thereby be isolated in the spot nanowire spaces. The water-absorbing material comes into contact with part of the aqueous solution on the surface of the substrate and absorbs the aqueous solution. In this manner, the aqueous solution spread over the entire substrate is gradually absorbed by the water-absorbing material. In this case, the aqueous solution in the hydrophobic region is more easily removed. However, the aqueous solution in the hydrophilic nanowire regions remains present in these regions. In this manner, the aqueous solution is isolated in the nanowire regions and remains therein.

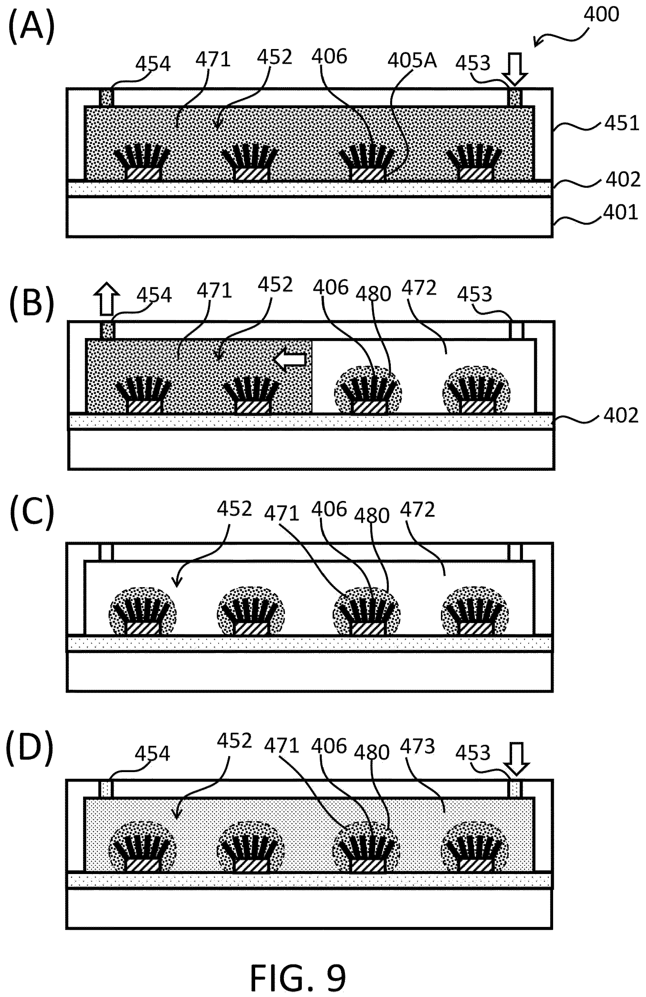

Fig. 9 is used to explain a method for isolating the aqueous solution into the spot nanowire spaces or forming microdroplets in an embodiment.

A flow channel device 400 shown in Fig. 9A is provided. The flow channel device 400 includes a first substrate 401 and a second substrate 451 that are joined together, and a flow channel space 452 is thereby formed inside the flow channel device 400. The first substrate 401 includes a hydrophobic film 402 disposed over the entire surface thereof. A plurality of nanowire regions 405A each including a plurality of nanowires 406 are disposed on the hydrophobic film 402. These are disposed inside the flow channel space 452. The second substrate 451 has a liquid inlet 453 and a liquid outlet 454. An aqueous solution 471 is introduced through the inlet 453 into the flow channel space 452 so as to fill the flow channel space 452 containing the nanowires 406, and an excess amount of the aqueous solution is discharged through the outlet 454.

As shown in Fig. 9B, the aqueous solution 471 is discharged from the flow channel space 452 to the outside through the outlet 454. In some embodiments, a water-absorbing material (not shown) may be brought into contact with the aqueous solution 471 at the outlet 454 to absorb the aqueous solution 471. As the aqueous solution 471 is discharged through the outlet 454, air 472 is introduced through the inlet 453 into the flow channel space 452. As the air 472 is introduced, the aqueous solution 471 on the hydrophobic surface 402 disappears, and the aqueous solution 471 remaining in spot nanowire spaces 480 restricted by the nanowires 406 forms droplets.

As shown in Fig. 9C, by discharging a sufficient or excess amount of the aqueous solution 471 from the flow channel space 452, the aqueous solution 471 is isolated only in the spot nanowire spaces 480 defined by the nanowires 406 within the flow channel space 452. A portion of the flow channel space 452 other than the spot nanowire spaces 480 is occupied by the air 472.

In the embodiment shown in Figs. 9A to 9C, the hydrophobic region 402 on the surface of the substrate 401 and the plurality of hydrophilic spot nanowire spaces 480 surrounded by the hydrophobic region 402 are used to isolate the aqueous solution 471 into the spot nanowire spaces 480, and so-called microdroplets can thereby be formed.

### <Other examples of isolation>

In some embodiments, a gas may be introduced onto the surface of the substrate, and the aqueous solution may be removed from the hydrophobic surface with the flow, pressure, force, etc. of the gas. In this case, the aqueous solution present in the hydrophobic region can be more easily removed. However, the aqueous solution present in the hydrophilic nanowire regions remains present in these regions. Alternatively, a gas may be introduced onto the surface of the substrate under the condition that the aqueous solution can be removed from the hydrophobic surface while the aqueous solution remains in the spot nanowire spaces. In this manner, the aqueous solution can be isolated in the spot nanowire spaces.

In some embodiments, a non-aqueous solution (which is used herein interchangeably with a non-water-based solution and a non-water-based liquid) may be introduced into the flow channel space 452. As shown in Fig. 9D, a non-aqueous solution 473 is introduced through the inlet 453 into the flow channel space 452. The non-aqueous solution 473 gradually fills the flow channel space 452 toward the outlet 454 and eventually fills the entire flow channel space 452 (excluding the nanowire spaces 480). An excess amount of the non-aqueous solution 473 may be discharged through the outlet 454. In this state, substantially all the nano-spot spaces 480 in the flow channel space 452 hold the aqueous solution 471 and are enclosed by the non-aqueous solution 473.

In the embodiment shown in Fig. 9D, the isolated aqueous solution 471 is enclosed within the spot nanowire spaces 480, with the non-aqueous solution 473 surrounding the aqueous solution 471. In this case, for example, the evaporation of the aqueous solution 471 can be prevented, and stable measurement or observation can be performed.

### <Introduction of non-aqueous solution>

In the embodiment in Fig. 9, the aqueous solution 471 present in the portion other than the spot nanowire spaces 480 is replaced with air. In some embodiments, the step of replacement with air shown in Figs. 9B to 9C may be changed to replacement with the non-aqueous solution. The non-aqueous solution may be a type of oil. In this case, the aqueous solution in the hydrophobic region is replaced with the introduced non-aqueous solution. However, the non-aqueous solution is unlikely to enter the hydrophilic spot nanowire spaces 480. Therefore, the aqueous solution in the hydrophilic spot nanowire spaces 480 are unlikely to be replaced with the non-aqueous solution. The aqueous solution is thereby isolated in the nanowire regions and remains therein. Moreover, the spot nanowire spaces holding the aqueous solution can be enclosed by the non-aqueous solution.

### <Isolation of target substance into droplets>

In some embodiments, an aqueous solution containing the target substance (such as target molecules) may be introduced onto the surface of the substrate, and then the aqueous solution may be removed from the hydrophobic surface. In this manner, the target substance can be isolated in the spot nanowire spaces.

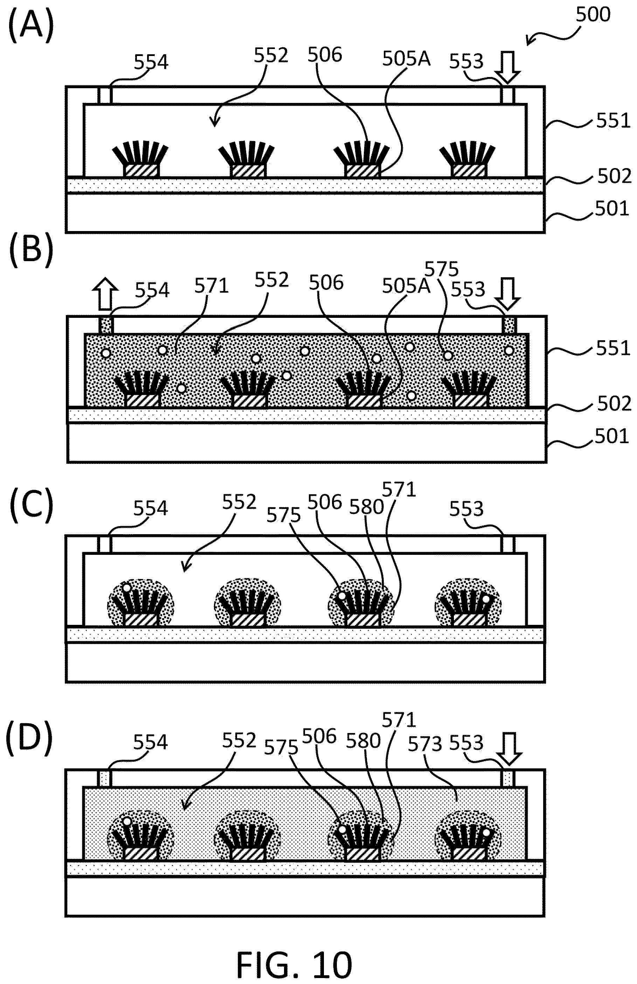

Referring to Fig. 10, a method for isolating the target substance into the spot nanowire spaces in an embodiment will be described.

A flow channel device 500 shown in Fig. 10A is provided. The flow channel device 500 includes a first substrate 501 and a second substrate 551 that are joined together, and a flow channel space 552 is thereby formed inside the flow channel device 500. The first substrate 501 includes a hydrophobic film 502 disposed over the entire surface thereof. A plurality of nanowire regions 505A each including a plurality of nanowires 506 are disposed on the hydrophobic film 502. These are disposed inside the flow channel space 552. The second substrate 551 has a liquid inlet 553 and a liquid outlet 554.

An aqueous solution 571 containing a target substance 575 is introduced through the inlet 553 into the flow channel space 552 to fill the flow channel space 552 including the nanowires 506, and an excess amount of the aqueous solution 571 is discharged through the outlet 554 (Fig. 10B).

Any of the methods in the embodiments of the disclosure or another method is used to isolate the aqueous solution 571 into the spot nanowire spaces 580 or as the spot nanowire spaces 580. In this case, the target substance 575 can be isolated in the spot nanowire spaces 580 (Fig. 10C).

In some embodiments, a non-aqueous solution may be further introduced into the flow channel space 552. As shown in Fig. 10D, the non-aqueous solution 573 is introduced through the inlet 553 into the flow channel space 552. The non-aqueous solution 573 gradually fills the flow channel space 552 toward the outlet 554 and eventually fills the entire flow channel space 552 (excluding the nanowire spaces 580). In this state, substantially all the nano-spot spaces 580 in the flow channel space 552 hold the aqueous solution 571 and the target substance 575 and are enclosed by the non-aqueous solution 573.

In some embodiments, the target substance may be captured on the nanowires. In Figs. 10C and 10D, the target substance 575 is captured on the surfaces of the nanowires 506 in the spot nanowire spaces 580. In some embodiments, the target substance is not captured on the nanowires but may be confined in the nanowire spaces.

In the embodiment shown in Fig. 10D, the isolated aqueous solution 571 and the target substance 575 contained therein are confined in the spot nanowire spaces 580, with a non-aqueous solution 572 surrounding the aqueous solution 571. In this manner, for example, the evaporation of the aqueous solution 571 can be prevented, and stable measurement or observation can be performed.

### <Example 1 of isolation of target substance>

In this Example, extracellular vesicles (EVs) were captured in nanowire spaces. Specifically, an aqueous solution containing EVs (2.3 × 10⁹ particles/mL) used as a target substance and EVs in which mCherry serving as a fluorescent protein marker recognizing the EVs was expressed was prepared. This aqueous solution was introduced into a device of the same type as that shown in Fig. 8.

In this Example, after the introduction of the aqueous solution into the device, the aqueous solution was incubated for 2 hours to bind mCherry to the EVs. In this case, it is considered that the capture of the EVs on the nanowires is also facilitated. Then the aqueous solution in the flow channel was absorbed from the discharge port using a water-absorbing sheet, and the flow channel of the device was washed with PBS. Droplets were thereby formed, and oil was introduced into the flow channel to protect the droplets.

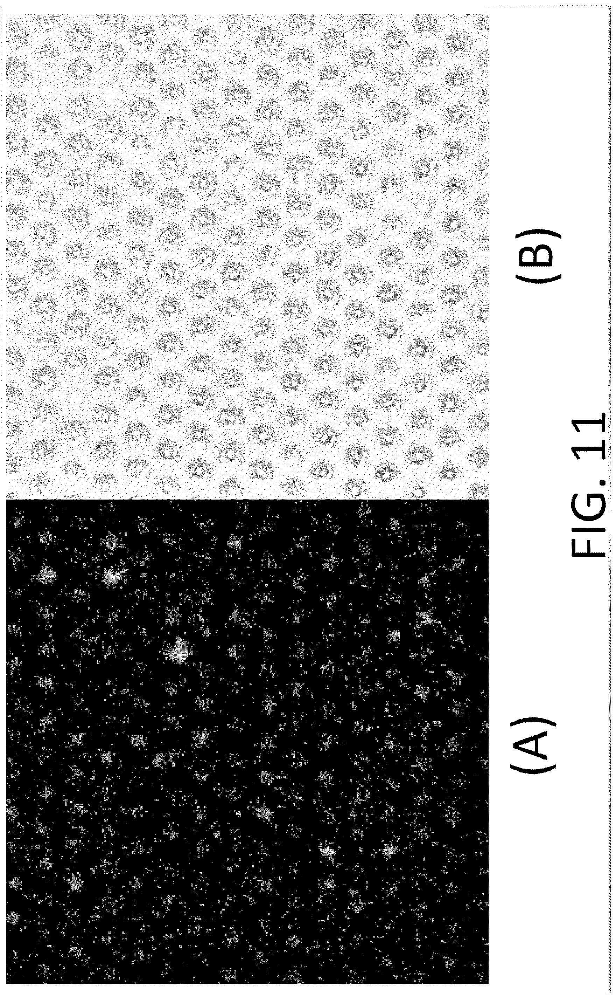

Fig. 11 shows a fluorescence microscope image (A) and a normal optical microscope image (B) viewed from above. In the optical microscope image (B), the positions of the spot nanowire spaces are identified. In the fluorescence microscope image (A), the fluorescence from mCherry is found only in the positions of the spot nanowire spaces. This shows that the EVs were captured and isolated in the spot nanowire spaces.

Other methods for isolating the target substance will be described.

### <Example 2 of isolation of target substance>

An aqueous solution was prepared using the same procedure as in Example 1. In this Example, after the introduction of the aqueous solution into the device, the aqueous solution in the flow channel was first absorbed through the discharge port using a water-absorbing sheet, and the remaining aqueous solution was incubated for 2 hours. Then the flow channel of the device was washed with PBS. Droplets were thereby formed, and oil was introduced into the flow channel to protect the droplets.

In a fluorescence microscope image, the EVs were found to be isolated and captured on the spot nanowire spaces, as in Example 1.

### <Example 3 of isolation of target substance>

An aqueous solution was prepared using the same procedure as in Example 1. In this Example, after the introduction of the aqueous solution into the device, the device was held in a vacuum desiccator for 2 hours. In this manner, water in the aqueous solution was evaporated, and the remaining aqueous solution was isolated in the spot nanowire spaces. Then the flow channel of the device was washed with PBS, and droplets were thereby formed. Oil was introduced into the flow channel to protect the droplets.

In a fluorescence microscope image, the EVs were found to be isolated and captured on the spot nanowire spaces, as in Example 1.

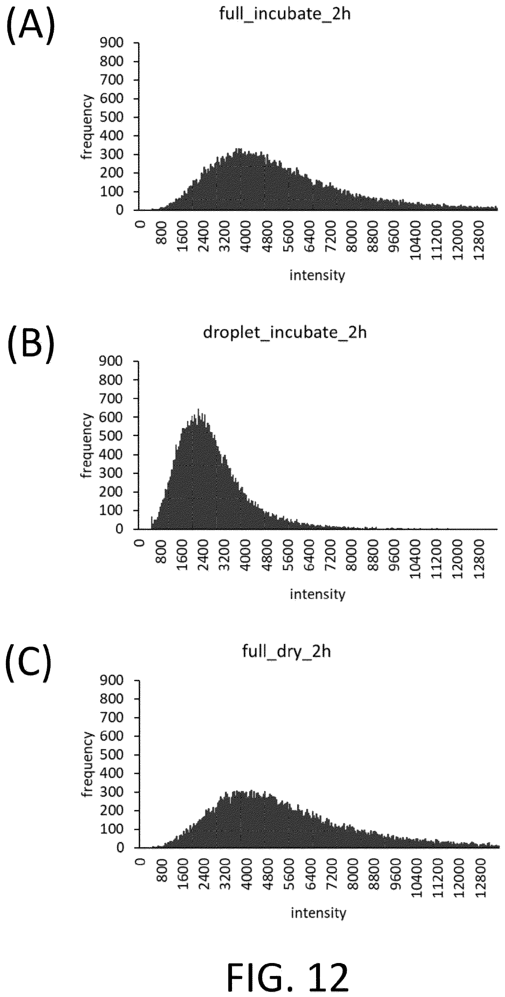

Fig. 12 shows the fluorescence intensity (the horizontal axis) at positions corresponding to the spot nanowire spaces in each of the fluorescence microscope images in Examples 1 to 3 above versus its frequency (the vertical axis).

The frequency distribution in Fig. 12A (Example 1) extends over a higher fluorescence intensity range than that in Fig. 12B (Example 2). This indicates that a larger amount of the fluorescent marker is present per spot. In other words, this indicates that the concentration of the fluorescent marker per spot nanowire space is higher. Specifically, the efficiency of capturing the EVs used as the target substance is higher.

In Example 2, when the aqueous solution was removed by absorption, the target substance and the fluorescent marker contained in the aqueous solution were also discharged from the flow channel. However, in Example 1, the target substance and the fluorescent marker were captured on the nanowire during incubation, and this may contribute to the broadening of the distribution in a high fluorescence intensity range in Fig. 12A.

The frequency distribution in Fig. 12C (Example 3) also extends over a higher fluorescence intensity range than that in Fig. 12B (Example 2). This also indicates that a larger amount of the fluorescent marker is present per spot. In other words, this indicates that the concentration of the fluorescent marker per spot nanowire space is higher. Specifically, the efficiency of capturing the EVs used as the target substance is higher.

In Example 3, the concentration of the target substance increased as the water evaporated by drying, so that the concentration was highest in the aqueous solution finally remaining in the spot nanowire spaces. Therefore, it may be considered that the target substance and the fluorescent marker captured at high concentrations in the spot nanowire spaces contribute to the broadening of the distribution in a high fluorescence intensity range.

The distribution in Fig. 12C is higher than that in Fig. 12A in a high fluorescence intensity range, but no large difference is found. **In** both of Figs. 12A and 12C, the target substance was isolated and captured in the spot nanowire spaces more efficiently than that in Fig. 12B (Example 2).

### <Quantitative analysis using digital droplet array>

A digital droplet array (nano-spot space array) in an embodiment of the disclosure may be used to perform quantitative analysis. The substrate, flow channel device, and method of the disclosure can be applied to a digital assay. **In** some embodiments, the aqueous solution to be introduced may contain a measurement target substance. The aqueous solution may have been subjected to extinction dilution or limiting dilution (it should be understood that the extinction dilution and the limiting dilution are not strictly defined and distinguished from each other and are not always used in a mutually exclusive manner). When the aqueous solution is introduced and isolated, the measurement target substance is confined in the nano-spot spaces.

In some embodiments, the measurement target substance may contain an optical or fluorescent label (which may be hereinafter referred to simply as an optical label) or may be bound to the optical or fluorescent label. In some embodiments, the measurement target substance may be bound to the optical or fluorescent label before introduction into the fluid device. In some embodiments, the measurement target substance may be bound to the optical or fluorescent label after introduction into the fluid device but before or after the isolation of the aqueous solution. For example, a first aqueous solution may contain the measurement target substance, and a second aqueous solution may contain the optical label. Then the first aqueous solution and the second aqueous solution may be separately introduced into the fluid device in any order. In this case, the measurement target substance may be bound to the optical or fluorescent label within the spot spaces (before or after the isolation).

In some embodiments, the measurement target substance and the optical label or a conjugate thereof may be captured on the nanowires. The phrase "captured" means that the measurement target substance and the optical label or the conjugate thereof adsorbs or is bound to the nanowires through a hydrogen bond, van der Waals force, an ionic bond, a covalent bond, etc., but it is not always necessary to identify its mechanism. The nanowires are known to capture various biomolecules or biological substances. Therefore, the spot spaces including the nanowires capture and isolate a biological substance efficiently, and the digital quantification of the biological substance is thereby facilitated.

### <Digital droplet array measurement: Example 1 (digital immunoassay)>

A digital droplet array in one embodiment of the disclosure can be used to perform a single-molecule digital immunoassay. Zero or one molecule is confined in each nano-spot space, and the digital droplet array (nano-spot space array) for identifying the molecules can thereby be produced.

In one example, the inventors have found that biotin can be used to measure the concentration of a complex (conjugate) of biotin and an anti-CD9 antibody by using a series of concentrations (not shown). 90 µL of one of the conjugate solutions was introduced into the flow channel device described above, and the device was left to stand at room temperature for 1 hour. Then the device was washed sufficiently.

Next, a 10 µg/mL streptavidin-β-galactosidase conjugate was prepared as a marker for biotin. 90 µL of the conjugate solution was introduced into the flow channel device, and the device was left to stand at room temperature for 1 hour. Then the device was washed sufficiently.

Then 10 µM Tokyo Green (registered trademark, the same applies to the following) was prepared as a fluorescent substrate for detecting β-galactosidase. 90 µL of the solution was introduced into the flow channel device.

Then 90 µL of an oil (product name FC40, 3M (registered trademark), Fluorinert (registered trademark)) was introduced into the above device to isolate the solution having undergone the reaction into the spot nanowire spaces, and the resulting device was left to stand overnight.

Then fluorescence microscope observation was performed, and the availability of the digital immunoassay was confirmed.

### <Digital droplet array measurement: Example 2 (ddPCR)>

A digital droplet array in one embodiment of the disclosure can be used to perform digital PCR (polymerase chain reaction), i.e., digital droplet PCR (ddPCR). The PCR may be real-time PCR (qPCR).

First, a sample nucleic acid is mixed with a primer and a probe. The solution mixture is introduced into the nano-spot space array device. Next, an oil is introduced into the device to isolate the solution mixture into the nano-spot spaces. PCR amplification is performed in each of the nano-spot spaces. Fluorescence from each nano-spot space is measured. The measurement results are used to determine whether each nano-spot space is positive or negative. The number of nano-spot spaces judged to be positive is counted to perform absolute quantification. The concentration (copies/µL) of the sample nucleic acid can be finally determined.

### <Digital droplet array measurement: Example 3 (digital ELISA)>

In an indirect method, a primary antibody is reacted with a target protein, and then a secondary antibody labeled with an enzyme to the primary antibody is allowed to react. The resulting conjugate is introduced into the nano-spot space array device. Next, a substrate is introduced into the device. Then the nano-spot spaces are isolated. The absorbance of each of the nano-spot spaces is measured. The measurement results are used to determine whether each nano-spot space is positive or negative. The number of nano-spot spaces judged to be positive is counted to perform absolute quantification. The concentration of the target protein can be finally determined. (See: Kim SH, et al., "Large-scale femtoliter droplet array for digital counting of single biomolecules," Lab on a Chip Online Edition: 2012/8/15 (Japan time), doi: 10.1039/C2LC40632B.)

The procedure for mixing the substance, the solution, etc. is not limited to the above procedure. For example, the primary antibody may be reacted with the antigen, and then the secondary antibody may be reacted with the resulting antigen. Then the enzyme may be introduced into the resulting antigen. Alternatively, another procedure may be used.

An example of the detection of a target miRNA in EVs will be described.

First, lipid nanoparticles are prepared. Molecular beacons having a sequence complementary to the sequence of the target miRNA are incorporated into the lipid nanoparticles with positively charged surfaces. Then NH₂-PEG molecules are embedded in membranes of the lipid nanoparticles.

The surfaces of the nanowires in the nano-spot spaces are activated, and the lipid nanoparticles are bound to the nanowires. For example, the surfaces of the nanowires are activated with N-hydroxysuccinimide (NHS). NH₂ is covalently bonded to the nanowires, and the lipid nanoparticles can thereby be immobilized on the nanowires through amide bonds. Specifically, a NW-CONH-lipid nanoparticle structure is prepared.

Target exosomes are introduced into the nano-spot spaces to form droplets. In this manner, the exosomes and the lipid nanoparticles are fused in each nano-spot space. As a result, the target miRNA in the exosomes and the molecular beacons in the lipid nanoparticles are reacted with each other. When the target miRNA is present inside the exosomes, the complementary nucleic acid sequence of the molecular beacons is bound to the nucleic acid sequence of the target miRNA, and the fluorescent dye of the molecular beacons is separated from the quencher and emits light. The presence or absence of light emission from each of a large number of nano-spot spaces can be observed.

The digital droplet array of the present disclosure and the use of the digital droplet array are not limited to those described above and can be used for different measurement. For example, a protein binding assay can be performed.

In some embodiments, the nano-spot spaces in the present disclosure can be used as detection sites for a fluorescently labeled antigen. Various processes can be used. Non-limiting examples include the following.
1) First, a primary antibody is introduced into the nano-spot spaces, and then an antigen is introduced. A fluorescently labeled secondary antibody is introduced into the resulting nano-spot spaces.
2) First, a primary antibody is introduced into the nano-spot spaces, and then an antigen is introduced. Then a secondary antibody is introduced. A fluorescently labeled IgG is introduced into the resulting nano-spot spaces.
3) First, a primary antibody is introduced into the nano-spot spaces, and then an antigen is introduced. Then a secondary antibody is introduced. A fluorescent substrate is introduced into the resulting nano-spot spaces, and an enzyme reaction is observed.
4) First, an antigen is introduced into the nano-spot spaces, then a fluorescently labeled primary antibody is introduced.

In 1) to 3), the antigen may be introduced into the primary antibody.

In some embodiments, the nano-spot spaces in the present disclosure can be used as detection sites for the reaction of a reaction solution containing target molecules, a polymerase, and a primer introduced into the nano-spot spaces. Various processes can be used. Non-limiting examples include the following.
1) First, the target molecules are immobilized on the nanowires in the nano-spot spaces, and then a reaction solution containing the polymerase and the primer is introduced.
2) First, the polymerase is immobilized on the nanowires in the nano-spot spaces, and then a reaction solution containing the target molecules and the primer is introduced.
3) First, the primer is immobilized on the nanowires in the nano-spot spaces, and a reaction solution containing the target molecules and the polymerase is introduced.
4) First, the target molecules, the polymerase, and the primer are immobilized on the nanowires in the nano-spot spaces, and then a reaction solution is introduced.

In this case, the reaction may be controlled by adjusting the salt concentration.

### <Other embodiments>

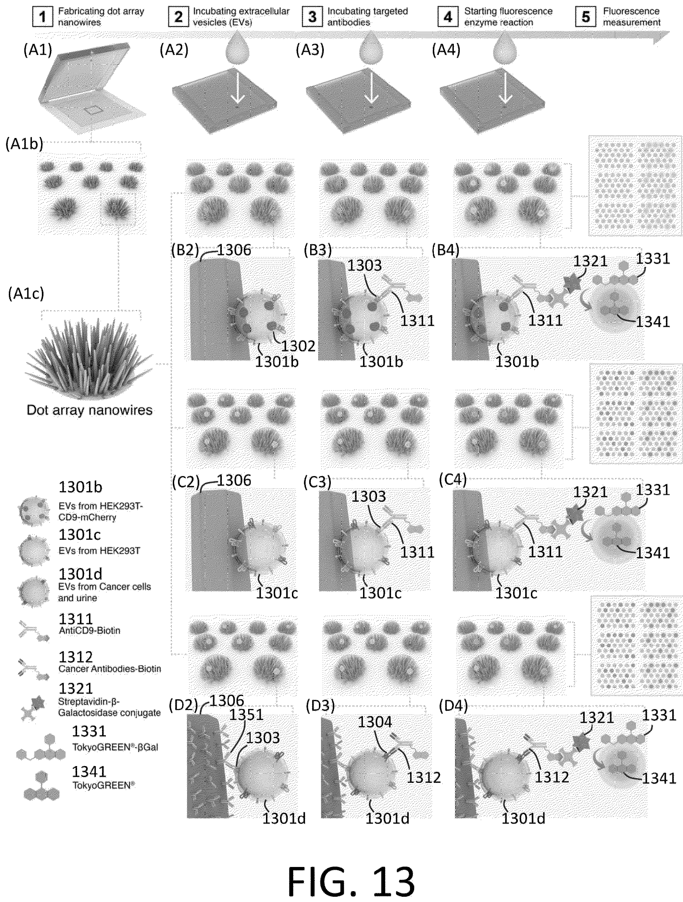

Referring to Fig. 13, some other embodiments will be described. Specific examples of the production of the nanowire device, capture of EVs, and fluorescence measurement will be described in the <Experimental method> section after the description related to Fig. 13 for the sake of readability.

A device including a nano-spot space array is provided (Fig. 13A1). A large number of nano-spot nanowires (which may be referred to also as dot array nanowires) (Fig. 13A1c) are arranged in an array in the flow channel of the device (Fig. 13A1b). Next, a target substance (EVs in Fig. 13) is introduced into the flow channel (Fig. 13A2). Then a targeted antibody is introduced into the flow channel and bound to the target substance (Fig. 13A3). A fluorescence enzyme reaction is started (Fig. 13A4), and fluorescence measurement is performed.

In one embodiment shown in Figs. 13B2 to 13B4, the target substance used may be EVs labeled with a fluorescent marker. In a non-limiting example in Fig. 13B, EVs 1301b which are derived from HEK293T cells, in which CD9 protein 1303 is expressed, and which are labelled with mCherry 1302 are used (the EVs are hereinafter referred to as "HEK293T-CD9-mCherry cell-derived EVs").

The HEK293T-CD9-mCherry cell-derived EVs 1301b introduced into the flow channel adsorb to nanowires 1306 (Fig. 13B2). Next, an anti-CD9-biotin conjugate 1311 used as a targeted antibody is introduced into the flow channel and recognizes the CD9 protein 1303 on the surfaces of the EVs 1301b, and an antigen-antibody conjugate is formed (Fig. 13B3). An avidin conjugate 1321 (a streptavidin-β-galactosidase conjugate in Fig. 13) is bound to the biotin in the biotin conjugate 1311 through the avidin-biotin interaction. Next, fluorescent substrates (TokyoGreen (Goryo Chemical, Inc.) in Fig. 13) 1331 and 1341 are introduced to measure the fluorescence intensity of each nano-spot (Fig. 13B4). In the optical measurement, both the fluorescence intensity of mCherry and the fluorescence intensity of TokyoGreen are measured.

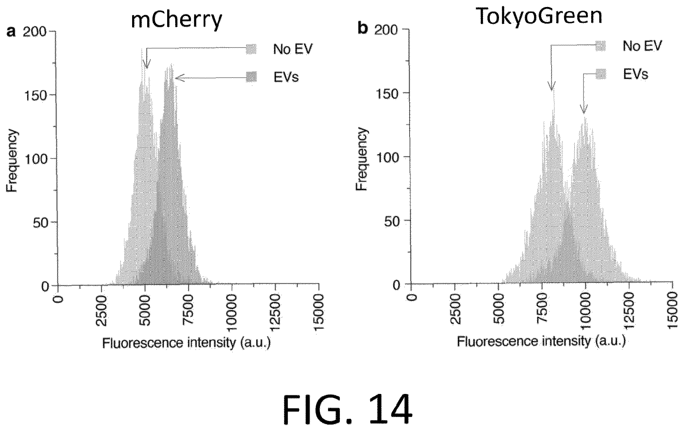

The fluorescence intensity actually obtained based on the protocol described above is shown in Fig. 14. In the case of mCherry (Fig. 14a) and also in the case of TG (Fig. 14b), the fluorescence intensity was found to be significantly higher than that in a negative control containing no EVs. The positions of spots of mCherry observed under a fluorescence microscope were almost the same as the positions of spots of TG. This indicates that the target EVs can be observed using TG.

In one embodiment shown in Figs. 13C2 to 13C4, a (non-labeled) target substance not labeled with a fluorescent marker etc. may be used. In a non-limiting example shown in Fig. 13C, EVs 1301c which are derived from HEK293T cells and in which CD9 protein 1303 is expressed are used (the EVs are hereinafter referred to as "HEK293T-CD9 cell-derived EVs").

The HEK293T-CD9 cell-derived EVs 1301c introduced into the flow channel adsorb to the nanowires 1306 (Fig. 13B2). Next, an anti-CD9-biotin conjugate 1311 used as a targeted antibody is introduced into the flow channel and recognizes the CD9 protein 1303 on the surfaces of the EVs 1301c, and an antigen-antibody conjugate is formed (Fig. 13C3). An avidin conjugate 1321 is bounded to the biotin in the biotin conjugate 1311 through the avidin-biotin interaction. Next, fluorescent substrates 1331 and 1341 are introduced to measure the fluorescence intensity of each nano spot (Fig. 13C4). In the optical measurement, the fluorescence intensity of TokyoGreen is measured.

In one embodiment shown in Figs. 13D2 to 13D4, an antibody that recognizes a protein including the target substance may be disposed on the surfaces of the nanowires 1306. In one non-limiting example, an anti-CD9 antibody 1351 may be immobilized on the surfaces of the nanowires 1306 (Fig. 13D2).

HEK293T-CD9 cell-derived EVs 1301d introduced into the flow channel approach the surfaces of the nanowires 1306, and the CD9 and the anti-CD9 antibody 1351 on the surfaces form an antigen-antibody conjugate (Fig. 13C2). Next, an anti-CD9-biotin conjugate 1311 used as a targeted antibody is introduced into the flow channel and recognizes the CD9 protein 1303 on the surfaces of the EVs 1301c, and an antigen-antibody conjugate is formed (Fig. 13C3). An avidin conjugate 1321 is bound to the biotin in the biotin conjugate 1311 through the avidin-biotin interaction. Next, fluorescent substrates 1331 and 1341 are introduced to measure the fluorescence intensity of each nano spot (Fig. 13C4). In the optical measurement, the fluorescence intensity of TokyoGreen is measured.

### <EXAMPLES>

The detection of high-grade serous ovarian carcinoma (HGSOC), a subtype of epithelial ovarian cancer (EOC), was attempted as an example of the embodiment shown in Figs. 13D2 to 13D4. As membrane proteins expressed on the surfaces of EVs derived from HGSOC cells, two membrane proteins were selected, including Claudin-3 (CLDN3) and Tumor-associated calcium signal transducer 2 (TACSTD2) whose gene expression in EOC tissues is known to be high.

The surfaces of the nanowires were modified with the anti-CD9 antibody. Urine samples were collected from HGSOC patients and non-cancer subjects (N = 5 for each case). The urine samples were appropriately prepared and introduced into devices. Next, a fluorescently labeled CLDN3 antibody and a fluorescently labeled TACSTD2 antibody were introduced into the devices. Other or detailed experimental conditions are shown below.

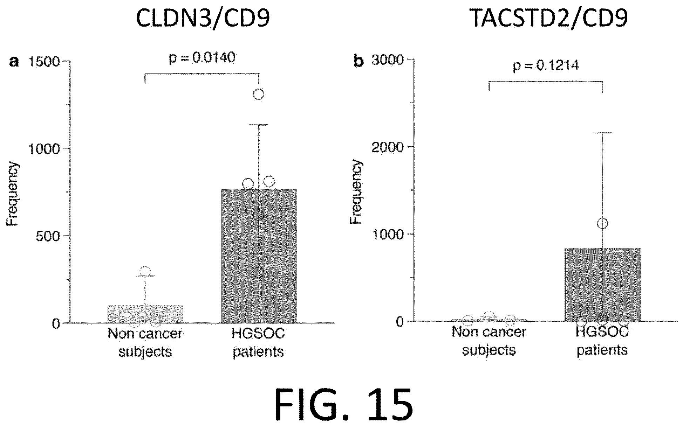

The results are shown in Fig. 15. The maximum value of the fluorescence intensity distribution (the maximum value of the Gaussian distribution) in a negative control with "no EVs" was used as a threshold value, and the frequency of the intensity higher than the threshold value was plotted. The results clearly show that the HGSOC cells can be detected using CD9 and CLDN3 as target proteins. The HGSOC cells were not detected in all the samples using the CD9 and TACSTD2 as target proteins. However, the results show the possibility of detection using these target proteins.

In the present Example, the EVs were used as the target substance. The anti-CD9 antibody was immobilized on the surfaces of the nanowires, and the fluorescently labeled CLDN3 antibody or the fluorescently labeled TACSTD2 antibody was used. However, the embodiment of the present disclosure is not limited thereto. In the present Example, the EVs were used as the target substance. However, different biomolecules or a different target substance may be used. In the present Example, these two proteins were detected, but the present disclosure is not limited thereto. A different membrane protein may be selected as a detection target. The antibody immobilized on the nanowires and the membrane protein of the target biomolecules to be captured on the nanowires may be appropriately selected. Similarly, the targeted antibody and the membrane protein of the target biomolecules recognized by the targeted antibody may also be appropriately selected. Generally, a plurality of antibodies that recognize the target substance can be used. The first antibody may be immobilized on the surfaces of the nanowires, and the introduced target substance may be bound to the immobilized first antibody. The second antibody may be fluorescently labeled and bound to the target substance. The target substance may be bound to the first antibody then immobilized on the surfaces of the nanowires and may form a conjugate bound to the fluorescently labeled second antibody. The conjugate in this state may be subjected to fluorescence reaction to detect the fluorescence. The fluorescent substrate, the fluorescently labeled primary and second antibodies may be selected appropriately.

The capture of the EVs in Figs. 13B2 and 13C2 is through to be based on the charge reaction between the EVs 1301b and the nanowires 1306. The capture of the EVs in Fig. 13D2 is thought to be based on the immunoaffinity between the anti-CD9 antibody 1351 immobilized on the nanowires 1306 and the CD9 protein 1309 expressed on the surfaces of the EVs 1301b. However, these capturing mechanisms are not to be interpreted as limiting, and the proposal of other scientifically available mechanisms is not precluded.

### <Experimental methods>

### <Production of spot nanowire device>

In the above Examples, first, a CYTOP film was formed on a synthetic quartz substrate by spin coating, and ZnO seed regions were formed by patterning. The nanowires were synthesized on the ZnO seed by a hydrothermal method at 95°C for 1 hour using 30 mM zinc nitrate hexahydrate (Zn(NO₃)₂·6H₂O, Thermo Fisher Scientific K.K.) and hexamethylenetetramine (HMTA, C₆H₁₂N₄, Wako Pure Chemical Industries, Ltd.). A flow channel chamber was produced by attaching a cycloolefin polymer-made flow channel block to the synthesized nanowire substrate.

### <Immobilization of anti-CD9 antibody on nanowires>

The flow channel of the device was filled with a 1 mg/mL anti-human CD9 antibody (Cosmo Bio Co., Ltd.), and the device was left to stand for 1 hour. Then the flow channel was washed with PBS. In this manner, the surfaces of the nanowires were modified with the anti-human CD9 antibody.

### <Preparation of urine samples>

Urine samples were collected from HGSOC patients and non-cancer subjects. Then the urine samples were subjected to centrifugation (10 minutes, 4°C, 300 × g and then 10 minutes, 4°C, 3000 × g) to remove apoptotic bodies and then filtered using a 0.22 µm filter (Merck Millipore Ltd.). Next, the filtered urine samples were incubated using a well plate assay system according to its procedure.

### <Capture of EVs>

The prepared urine samples were introduced in an amount of 6 µL into the devices. Next, a blocking solution (KPL 10% BSA blocking solution, SeraCare) was introduced, and the resulting samples were incubated at 37°C for 1 hour. Then PBS was caused to flow to wash out the remaining samples.

### <Recognition by targeted antibody>

A biotin-labeled anti-human CLDN3 antibody (OriGene Technologies, Inc.) or a biotinylated anti-human TACSTD2 antibody (Exbio Praha a.s.) was used as the primary antibody and introduced at 100 nM into each device. Incubation was performed at room temperature for 1 hour, and the device was washed three times with PBS. Next, streptavidin-β gal (SIGMA) was used for enzyme labelling and introduced at 1 nM into each device. Incubation was performed at room temperature for 1 hour, and the device was washed three times with PBS. To allow the enzyme reaction to proceed on the dot array nanowires, the channel was filled with a 10 µM substrate TokyoGreen (TG) (Goryo Chemical, Inc.), and the TG solution in the channel was replaced with FC-40 (3M Japan). Then incubation was performed at 37°C for 20 hours.

### <Fluorescence observation>

Fluorescence imaging was performed using a confocal microscope (Leica Microsystems) for observation. 1024 × 1024 pixel 16 bit images were taken. Fluorescence images of mCherry were captured by excitation at 561 nm and 600 to 800 nm, and fluorescence images of TG were captured by excitation and light emission at 488 nm and 500 to 550 nm. AquaCOSMOS software (Hamamatsu Photonics K.K.) was used to analyze the fluorescence intensity from the dot array nanowires (spot nanowires).

The present disclosure also provides the following embodiments.

### A001

A method for processing an aqueous solution and/or a method for forming microdroplets, the method including:
providing a substrate/device/flow channel having a surface including a hydrophobic surface (region) and a region (nanowire region) surrounded by the hydrophobic surface, wherein the nanowire region includes hydrophilic nanowires;
introducing an aqueous solution onto the surface of the substrate; and
isolating the aqueous solution into the nanowire region.

### A002

The method according to A001 or any of the embodiments,
wherein the nanowire region includes a plurality of nanowire regions each surrounded by the hydrophobic surface.

### A011

The method according to A001 or A002 or any of the embodiments,
wherein the isolating the aqueous solution into the hydrophilic nanowires includes removing the introduced aqueous solution from the hydrophobic surface.

### A012a

The method according to A001 or any of the embodiments,
wherein the isolating the aqueous solution into the hydrophilic nanowires includes causing a water-absorbing material to absorb the aqueous solution on the hydrophobic surface.

### A012b

The method according to A011 or any of the embodiments,
wherein the isolating the aqueous solution into the hydrophilic nanowires includes introducing a gas (for example, air) onto the surface of the substrate.

### A012c

The method according to A011, A012a, or A012b or any of the embodiments,
wherein the isolating the aqueous solution into the hydrophilic nanowires includes introducing a non-aqueous solution onto the surface of the substrate.

### A020

The method according to any one of A001 to A012c or any of the embodiments,
wherein the method for processing an aqueous solution and/or the method for forming microdroplets is a method for performing a digital assay.

### A021

The method according to A020 or any of the embodiments,
wherein the digital assay is selected from the group consisting of ddPCR (qPCR), digital ELISA, and a protein binding assay.

### A022

The method according to any one of A001 to A021 or any of the embodiments,
wherein the introducing the aqueous solution onto the surface of the substrate includes introducing a (first) aqueous solution containing a target substance.

### A023

The method according to A022 or any of the embodiments,
wherein the aqueous solution containing the target substance has been subjected to extinction dilution or limiting dilution.

### A025

The method according to A022 or A023 or any of the embodiments, further including, after the isolating the aqueous solution into the nanowire region, measuring or detecting the target substance present in the isolated nanowire region.

### A031

The method according to any one of A022 to A025 or any of the embodiments, further including
capturing the target substance on the hydrophilic nanowires.

### A032

The method according to A031 or any of the embodiments, further including
detecting the target substance captured on the hydrophilic nanowires.

### A041

The method according to any one of A022 to A032 or any of the embodiments,
wherein the aqueous solution includes a first aqueous solution containing the target substance and a second aqueous solution containing a marker that recognizes the target substance.

### A042

The method according to any one of A022 to A032 or any of the embodiments,
wherein the introducing the aqueous solution onto the surface of the substrate includes introducing a first aqueous solution containing the target substance and introducing a second aqueous solution containing a marker that recognizes the target substance.

### A043

The method according to A041 or A042 or any of the embodiments, further including causing the target substance the marker to recognize the target substance.

### A044a

The method according to A041 or A042 or any of the embodiments, (further) including:
introducing the first aqueous solution containing the target substance to cause the nanowires to capture the target substance; and
introducing the second aqueous solution containing the marker that recognizes the target substance to cause the marker to recognize the target substance captured on the nanowires.

### A044b

The method according to A041 or A042 or any of the embodiments, (further) including:
introducing the second aqueous solution containing the marker that recognizes the target substance to cause the nanowires to capture the marker; and
introducing the first aqueous solution containing the target substance to cause the marker captured on the nanowires to recognize the target substance.

### A044c

The method according to A041 or A042 or any of the embodiments, (further) including:
introducing the first aqueous solution containing the target substance;
introducing the second aqueous solution containing the marker that recognizes the target substance;
causing the marker to recognize the target substance; and
causing the nanowires to capture a conjugate of the marker and the target substance.

### A051

The method according to any one of A041 to A044c or any of the embodiments, wherein the marker includes an optical marker (a fluorescent marker or a light-absorbing marker).

### A052

The method according to A051 or any of the embodiments, further including detecting the optical marker by an optical method.

### A101

A method for detecting biomolecules, including:
providing a device including a plurality of spot nanowire regions;
introducing target biomolecules onto surfaces of nanowires in the plurality of spot nanowire regions to capture the target biomolecules on the surfaces of the nanowires;
introducing a first antibody that is labeled with an optical label and recognizes a first membrane protein of the target biomolecules into the device to cause the first antibody to recognize the target biomolecules; and
optically measuring the optical label.

### A111

A method for detecting biomolecules, including:
providing a device including a plurality of spot nanowire regions;
disposing a second antibody that recognizes a second membrane protein of the target biomolecules on surfaces of nanowires in the plurality of spot nanowire regions;
introducing the target biomolecules into the plurality of spot nanowire regions to cause the second antibody to capture the target biomolecules;
introducing a first antibody that is labelled with an optical label and recognizes a first membrane protein of the target biomolecules into the device to cause the first antibody to recognize the target biomolecules; and
optically measuring the optical label.

### A121

The method according to A111 or any of the embodiments,
wherein the first membrane protein differs from the second membrane protein, and/or
the first antibody differs from the second antibody.

While the preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations, or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations, or equivalents. It is intended that the following claims define the scope of the invention and that methods and structures within the scope of these claims and their equivalents be covered thereby.

## Claims

1. A method for forming microdroplets, comprising:
providing a device having a substrate surface including a hydrophobic surface (region) and a plurality of regions (nanowire regions) surrounded by the hydrophobic surface, wherein the nanowire regions each include hydrophilic nanowires;
introducing an aqueous solution onto the substrate surface; and
isolating the aqueous solution into the nanowire regions.

2. The method according to claim 1,
wherein the isolating the aqueous solution into the hydrophilic nanowires includes removing the introduced aqueous solution from the hydrophobic surface.

3. The method according to claim 1,
wherein the isolating the aqueous solution into the hydrophilic nanowires includes causing a water-absorbing material to absorb the aqueous solution on the hydrophobic surface.

4. The method according to claim 1,
wherein the isolating the aqueous solution into the hydrophilic nanowires includes introducing a gas onto the substrate surface.

5. The method according to claim 1,
wherein the isolating the aqueous solution into the hydrophilic nanowires includes introducing a non-aqueous solution onto the substrate surface.

6. The method according to any one of claims 1 to 5,
wherein a method for processing the aqueous solution / the method for forming microdroplets includes performing a digital assay.

7. The method according to claim 6,
wherein the digital assay is selected from the group consisting of ddPCR (qPCR), digital ELISA, and a protein binding assay.

8. The method according to claim 1,
wherein the introducing the aqueous solution onto the substrate surface includes introducing a (first) aqueous solution containing a target substance.

9. The method according to claim 8, further comprising,
after the isolating the aqueous solution into the nanowire regions, measuring or detecting the target substance present in the isolated nanowire regions.

10. The method according to claim 8, further comprising
causing the hydrophilic nanowires to capture the target substance.

11. The method according to claim 10, further comprising
detecting the target substance captured on the nanowires.

12. The method according to claim 8,
wherein the aqueous solution includes a first aqueous solution containing the target substance and a second aqueous solution containing a marker that recognizes the target substance.

13. The method according to claim 8,
wherein the introducing the aqueous solution onto the substrate surface includes introducing a first aqueous solution containing the target substance and introducing a second aqueous solution containing a marker that recognizes the target substance.

14. The method according to claim 12, further comprising
causing the target substance the marker to recognize the target substance.

15. The method according to claim 12, (further) comprising:
introducing the first aqueous solution containing the target substance to cause the nanowires to capture the target substance; and
introducing the second aqueous solution containing the marker that recognizes the target substance to cause the marker to recognize the target substance captured on the nanowires.

16. The method according to claim 12, (further) comprising:
introducing the second aqueous solution containing the marker that recognizes the target substance to cause the nanowires to capture the marker; and
introducing the first aqueous solution containing the target substance to cause the marker captured on the nanowires to recognize the target substance.

17. The method according to claim 12, (further) comprising:
introducing the first aqueous solution containing the target substance;
introducing the second aqueous solution containing the marker that recognizes the target substance;
causing the marker to recognize the target substance; and
causing the nanowires to capture a conjugate of the marker and the target substance.

18. The method according to any one of claims 12 to 17 or any of the embodiments, wherein the marker includes an optical marker (a fluorescent marker or a light-absorbing marker).

19. The method according to claim 18, further comprising
detecting the optical marker by an optical method.

20. A method for detecting biomolecules, comprising:
providing a device including a plurality of spot nanowire regions;
disposing a second antibody that recognizes a second membrane protein of the target biomolecules on surfaces of nanowires in the plurality of spot nanowire regions;
introducing the target biomolecules into the plurality of spot nanowire regions to cause the second antibody to capture the target biomolecules;
introducing a first antibody that is labeled with an optical label and recognizes a first membrane protein of the target biomolecules into the device to cause the first antibody to recognize the target biomolecules; and
optically measuring the optical label.

21. The method according to claim 20,
wherein the first membrane protein differs from the second membrane protein, and/or
the first antibody differs from the second antibody.
